# EUROPEAN PATENT APPLICATION

(11) **EP 2 878 672 A1**
(43) Date of publication of application: **03.06.2015**
(21) Application number: 13822713.7
(22) Date of filing: 23.07.2013
(51) Int. Cl.: C12N 15/09, A61K 45/00, A61P 35/00, A61P 43/00, C07K 14/47, C12Q 1/68, G01N 33/53, G01N 33/574

(54) **FUSION GENE OF CEP55 GENE AND RET GENE**

(30) Priority: 26.07.2012 JP 2012165694
(71) Applicant: National Cancer Center, Tokyo 1040045 (JP); LSIP, LLC, Chiyoda-ku Tokyo 100-0005 (JP)
(72) Inventor: SHIBATA, Tatsuhiro, Tokyo 104-0045 (JP); HOSODA, Fumie, Tokyo 104-0045 (JP)
(74) Representative: Cabinet Plasseraud
(86) International application number: PCT/JP2013/069929
(87) International publication number: WO 2014/017491

(57) **Abstract**

In order to identify genes that can serve as indicators for predicting the effectiveness of drug treatments in cancers and provide novel methods for predicting the effectiveness of treatments with drugs targeting said genes, transcriptome sequencing was performed of diffuse-type gastric cancer. As a result, in-frame fusion transcripts between the CEP55 gene and the RET gene were identified. It was also found that said gene fusions induce activation of RET protein, thereby causing canceration of cells. Further, it was demonstrated that the RET protein activation and canceration caused by said gene fusion can be suppressed by using a RET tyrosine kinase inhibitor, and that treatments with a RET tyrosine kinase inhibitor are effective in patients with detection of said gene fusion.

SEQUENCE LISTING
<110>  NATIONAL CANCER CENTER
<120>  CEP55-RET FUSION GENE
<130>  IBPF13-538WO
<150>  JP2012/165694
<151>  2012-07-26
<160>  10
<170>  PatentIn version 3.1

<210>  1
<211>  2656
<212>  DNA
<213>  Homo sapiens
<220>
<221>  CDS
<222>  (305)..(1699)
<223>
<400>  1
   cacacctgat ggtgtgactc ggccgacgcg agcgccgcgc ttcgcttcag ctgctagctg      60
   gcccaaggga ggcgaccgcg gagggtggcg aggggcggcc aggacccgca gccccggggc     120
   cgggccggtc cggaccgcca gggagggcag gtcagtgggc agatcgcgtc cgcgggattc     180
   aatctctgcc cgctctgata acagtccttt tccctggcgc tcacttcgtg cctggcaccc     240
   ggctgggcgc ctcaagaccg ttgtctcttc gatcgcttct ttggacttgg cgaccatttc     300
   agag atg tct tcc aga agt acc aaa gat tta att aaa agt aag tgg gga      349
        Met Ser Ser Arg Ser Thr Lys Asp Leu Ile Lys Ser Lys Trp Gly
        1               5                   10                  15
   tcg aag cct agt aac tcc aaa tcc gaa act aca tta gaa aaa tta aag      397
   Ser Lys Pro Ser Asn Ser Lys Ser Glu Thr Thr Leu Glu Lys Leu Lys
                   20                  25                  30
   gga gaa att gca cac tta aag aca tca gtg gat gaa atc aca agt ggg      445
   Gly Glu Ile Ala His Leu Lys Thr Ser Val Asp Glu Ile Thr Ser Gly
               35                  40                  45
   aaa gga aag ctg act gat aaa gag aga cac aga ctt ttg gag aaa att      493
   Lys Gly Lys Leu Thr Asp Lys Glu Arg His Arg Leu Leu Glu Lys Ile
           50                  55                  60
   cga gtc ctt gag gct gag aag gag aag aat gct tat caa ctc aca gag      541
   Arg Val Leu Glu Ala Glu Lys Glu Lys Asn Ala Tyr Gln Leu Thr Glu
       65                  70                  75
   aag gac aaa gaa ata cag cga ctg aga gac caa ctg aag gcc aga tat      589
   Lys Asp Lys Glu Ile Gln Arg Leu Arg Asp Gln Leu Lys Ala Arg Tyr
   80                  85                  90                  95
   agt act acc gca ttg ctt gaa cag ctg gaa gag aca acg aga gaa gga      637
   Ser Thr Thr Ala Leu Leu Glu Gln Leu Glu Glu Thr Thr Arg Glu Gly
                   100                 105                 110
   gaa agg agg gag cag gtg ttg aaa gcc tta tct gaa gag aaa gac gta      685
   Glu Arg Arg Glu Gln Val Leu Lys Ala Leu Ser Glu Glu Lys Asp Val
               115                 120                 125
   ttg aaa caa cag ttg tct gct gca acc tca cga att gct gaa ctt gaa      733
   Leu Lys Gln Gln Leu Ser Ala Ala Thr Ser Arg Ile Ala Glu Leu Glu
           130                 135                 140
   agc aaa acc aat aca ctc cgt tta tca cag act gtg gct cca aac tgc      781
   Ser Lys Thr Asn Thr Leu Arg Leu Ser Gln Thr Val Ala Pro Asn Cys
       145                 150                 155
   ttc aac tca tca ata aat aat att cat gaa atg gaa ata cag ctg aaa      829
   Phe Asn Ser Ser Ile Asn Asn Ile His Glu Met Glu Ile Gln Leu Lys
   160                 165                 170                 175
   gat gct ctg gag aaa aat cag cag tgg ctc gtg tat gat cag cag cgg      877
   Asp Ala Leu Glu Lys Asn Gln Gln Trp Leu Val Tyr Asp Gln Gln Arg
                   180                 185                 190
   gaa gtc tat gta aaa gga ctt tta gca aag atc ttt gag ttg gaa aag      925
   Glu Val Tyr Val Lys Gly Leu Leu Ala Lys Ile Phe Glu Leu Glu Lys
               195                 200                 205
   aaa acg gaa aca gct gct cat tca ctc cca cag cag aca aaa aag cct      973
   Lys Thr Glu Thr Ala Ala His Ser Leu Pro Gln Gln Thr Lys Lys Pro
           210                 215                 220
   gaa tca gaa ggt tat ctt caa gaa gag aag cag aaa tgt tac aac gat      1021
   Glu Ser Glu Gly Tyr Leu Gln Glu Glu Lys Gln Lys Cys Tyr Asn Asp
       225                 230                 235
   ctc ttg gca agt gca aaa aaa gat ctt gag gtt gaa cga caa acc ata      1069
   Leu Leu Ala Ser Ala Lys Lys Asp Leu Glu Val Glu Arg Gln Thr Ile
   240                 245                 250                 255
   act cag ctg agt ttt gaa ctg agt gaa ttt cga aga aaa tat gaa gaa      1117
   Thr Gln Leu Ser Phe Glu Leu Ser Glu Phe Arg Arg Lys Tyr Glu Glu
                   260                 265                 270
   acc caa aaa gaa gtt cac aat tta aat cag ctg ttg tat tca caa aga      1165
   Thr Gln Lys Glu Val His Asn Leu Asn Gln Leu Leu Tyr Ser Gln Arg
               275                 280                 285
   agg gca gat gtg caa cat ctg gaa gat gat agg cat aaa aca gag aag      1213
   Arg Ala Asp Val Gln His Leu Glu Asp Asp Arg His Lys Thr Glu Lys
           290                 295                 300
   ata caa aaa ctc agg gaa gag aat gat att gct agg gga aaa ctt gaa      1261
   Ile Gln Lys Leu Arg Glu Glu Asn Asp Ile Ala Arg Gly Lys Leu Glu
       305                 310                 315
   gaa gag aag aag aga tcc gaa gag ctc tta tct cag gtc cag ttt ctt      1309
   Glu Glu Lys Lys Arg Ser Glu Glu Leu Leu Ser Gln Val Gln Phe Leu
   320                 325                 330                 335
   tac aca tct ctg cta aag cag caa gaa gaa caa aca agg gta gct ctg      1357
   Tyr Thr Ser Leu Leu Lys Gln Gln Glu Glu Gln Thr Arg Val Ala Leu
                   340                 345                 350
   ttg gaa caa cag atg cag gca tgt act tta gac ttt gaa aat gaa aaa      1405
   Leu Glu Gln Gln Met Gln Ala Cys Thr Leu Asp Phe Glu Asn Glu Lys
               355                 360                 365
   ctc gac cgt caa cat gtg cag cat caa ttg cat gta att ctt aag gag      1453
   Leu Asp Arg Gln His Val Gln His Gln Leu His Val Ile Leu Lys Glu
           370                 375                 380
   ctc cga aaa gca aga aat caa ata aca cag ttg gaa tcc ttg aaa cag      1501
   Leu Arg Lys Ala Arg Asn Gln Ile Thr Gln Leu Glu Ser Leu Lys Gln
       385                 390                 395
   ctt cat gag ttt gcc atc aca gag cca tta gtc act ttc caa gga gag      1549
   Leu His Glu Phe Ala Ile Thr Glu Pro Leu Val Thr Phe Gln Gly Glu
   400                 405                 410                 415
   act gaa aac aga gaa aaa gtt gcc gcc tca cca aaa agt ccc act gct      1597
   Thr Glu Asn Arg Glu Lys Val Ala Ala Ser Pro Lys Ser Pro Thr Ala
                   420                 425                 430
   gca ctc aat gaa agc ctg gtg gaa tgt ccc aag tgc aat ata cag tat      1645
   Ala Leu Asn Glu Ser Leu Val Glu Cys Pro Lys Cys Asn Ile Gln Tyr
               435                 440                 445
   cca gcc act gag cat cgc gat ctg ctt gtc cat gtg gaa tac tgt tca      1693
   Pro Ala Thr Glu His Arg Asp Leu Leu Val His Val Glu Tyr Cys Ser
           450                 455                 460
   aag tag caaaataagt atttgttttg atattaaaag attcaatact gtattttctg      1749
   Lys
   ttagcttgtg ggcattttga attatatatt tcacattttg cataaaactg cctatctacc      1809
   tttgacactc cagcatgcta gtgaatcatg tatcttttag gctgctgtgc atttctcttg      1869
   gcagtgatac ctccctgaca tggttcatca tcaggctgca atgacagaat gtggtgagca      1929
   gcgtctactg agactactaa cattttgcac tgtcaaaata cttggtgagg aaaagatagc      1989
   tcaggttatt gctaatgggt taatgcacca gcaagcaaaa tattttatgt tttggggttt      2049
   ttgaaaaatc aaagataatt aaccaaggat cttaactgtg ttcgcatttt ttatccaagc      2109
   acttagaaaa cctacaatcc taattttgat gtccattgtt aagaggtggt gatagatact      2169
   attttttttt tcatattgta tagcggttat tagaaaagtt ggggattttc ttgatcttta      2229
   ttgctgctta ccattgaaac ttaacccagc tgtgttcccc aactctgttc tgcgcacgaa      2289
   acagtatctg tttgaggcat aatcttaagt ggccacacac aatgttttct cttatgttat      2349
   ctggcagtaa ctgtaacttg aattacatta gcacattctg cttagctaaa attgttaaaa      2409
   taaactttaa taaacccatg tagccctctc atttgattga cagtatttta gttatttttg      2469
   gcattcttaa agctgggcaa tgtaatgatc agatctttgt ttgtctgaac aggtattttt      2529
   atacatgctt tttgtaaacc aaaaactttt aaatttcttc aggttttcta acatgcttac      2589
   cactgggcta ctgtaaatga gaaaagaata aaattattta atgttttaaa aaaaaaaaaa   2649
   aaaaaaa                                                            2656

<210>  2
<211>  464
<212>  PRT
<213>  Homo sapiens
<400>  2
Met Ser Ser Arg Ser Thr Lys Asp Leu Ile Lys Ser Lys Trp Gly Ser
1               5                   10                  15
Lys Pro Ser Asn Ser Lys Ser Glu Thr Thr Leu Glu Lys Leu Lys Gly
            20                  25                  30
Glu Ile Ala His Leu Lys Thr Ser Val Asp Glu Ile Thr Ser Gly Lys
        35                  40                  45
Gly Lys Leu Thr Asp Lys Glu Arg His Arg Leu Leu Glu Lys Ile Arg
    50                  55                  60
Val Leu Glu Ala Glu Lys Glu Lys Asn Ala Tyr Gln Leu Thr Glu Lys
65                  70                  75                  80
Asp Lys Glu Ile Gln Arg Leu Arg Asp Gln Leu Lys Ala Arg Tyr Ser
                85                  90                  95
Thr Thr Ala Leu Leu Glu Gln Leu Glu Glu Thr Thr Arg Glu Gly Glu
            100                 105                 110
Arg Arg Glu Gln Val Leu Lys Ala Leu Ser Glu Glu Lys Asp Val Leu
        115                 120                 125
Lys Gln Gln Leu Ser Ala Ala Thr Ser Arg Ile Ala Glu Leu Glu Ser
    130                 135                 140
Lys Thr Asn Thr Leu Arg Leu Ser Gln Thr Val Ala Pro Asn Cys Phe
145                 150                 155                 160
Asn Ser Ser Ile Asn Asn Ile His Glu Met Glu Ile Gln Leu Lys Asp
                165                 170                 175
Ala Leu Glu Lys Asn Gln Gln Trp Leu Val Tyr Asp Gln Gln Arg Glu
            180                 185                 190
Val Tyr Val Lys Gly Leu Leu Ala Lys Ile Phe Glu Leu Glu Lys Lys
        195                 200                 205
Thr Glu Thr Ala Ala His Ser Leu Pro Gln Gln Thr Lys Lys Pro Glu
    210                 215                 220
Ser Glu Gly Tyr Leu Gln Glu Glu Lys Gln Lys Cys Tyr Asn Asp Leu
225                 230                 235                 240
Leu Ala Ser Ala Lys Lys Asp Leu Glu Val Glu Arg Gln Thr Ile Thr
                245                 250                 255
Gln Leu Ser Phe Glu Leu Ser Glu Phe Arg Arg Lys Tyr Glu Glu Thr
            260                 265                 270
Gln Lys Glu Val His Asn Leu Asn Gln Leu Leu Tyr Ser Gln Arg Arg
        275                 280                 285
Ala Asp Val Gln His Leu Glu Asp Asp Arg His Lys Thr Glu Lys Ile
    290                 295                 300
Gln Lys Leu Arg Glu Glu Asn Asp Ile Ala Arg Gly Lys Leu Glu Glu
305                 310                 315                 320
Glu Lys Lys Arg Ser Glu Glu Leu Leu Ser Gln Val Gln Phe Leu Tyr
                325                 330                 335
Thr Ser Leu Leu Lys Gln Gln Glu Glu Gln Thr Arg Val Ala Leu Leu
            340                 345                 350
Glu Gln Gln Met Gln Ala Cys Thr Leu Asp Phe Glu Asn Glu Lys Leu
        355                 360                 365
Asp Arg Gln His Val Gln His Gln Leu His Val Ile Leu Lys Glu Leu
    370                 375                 380
Arg Lys Ala Arg Asn Gln Ile Thr Gln Leu Glu Ser Leu Lys Gln Leu
385                 390                 395                 400
His Glu Phe Ala Ile Thr Glu Pro Leu Val Thr Phe Gln Gly Glu Thr
                405                 410                 415
Glu Asn Arg Glu Lys Val Ala Ala Ser Pro Lys Ser Pro Thr Ala Ala
            420                 425                 430
Leu Asn Glu Ser Leu Val Glu Cys Pro Lys Cys Asn Ile Gln Tyr Pro

435                 440                 445
Ala Thr Glu His Arg Asp Leu Leu Val His Val Glu Tyr Cys Ser Lys
    450                 455                 460

<210>  3
<211>  5629
<212>  DNA
<213>  Homo sapiens
<220>
<221>  CDS
<222>  (191)..(3535)
<223>
<400>  3
agtcccgcga ccgaagcagg gcgcgcagca gcgctgagtg ccccggaacg tgcgtcgcgc      60
ccccagtgtc cgtcgcgtcc gccgcgcccc gggcggggat ggggcggcca gactgagcgc     120
cgcacccgcc atccagaccc gccggcccta gccgcagtcc ctccagccgt ggccccagcg     180
cgcacgggcg atg gcg aag gcg acg tcc ggt gcc gcg ggg ctg cgt ctg        229
ctg ttg ctg ctg ctg ctg ccg ctg cta ggc aaa gtg gca ttg ggc ctc       277
tac ttc tcg agg gat gct tac tgg gag aag ctg tat gtg gac cag gcg       325
gcc ggc acg ccc ttg ctg tac gtc cat gcc ctg cgg gac gcc cct gag       373
gag gtg ccc agc ttc cgc ctg ggc cag cat ctc tac ggc acg tac cgc       421
aca cgg ctg cat gag aac aac tgg atc tgc atc cag gag gac acc ggc       469
ctc ctc tac ctt aac cgg agc ctg gac cat agc tcc tgg gag aag ctc       517
agt gtc cgc aac cgc ggc ttt ccc ctg ctc acc gtc tac ctc aag gtc       565
ttc ctg tca ccc aca tcc ctt cgt gag ggc gag tgc cag tgg cca ggc       613
tgt gcc cgc gta tac ttc tcc ttc ttc aac acc tcc ttt cca gcc tgc       661
agc tcc ctc aag ccc cgg gag ctc tgc ttc cca gag aca agg ccc tcc       709
ttc cgc att cgg gag aac cga ccc cca ggc acc ttc cac cag ttc cgc       757
ctg ctg cct gtg cag ttc ttg tgc ccc aac atc agc gtg gcc tac agg       805
ctc ctg gag ggt gag ggt ctg ccc ttc cgc tgc gcc ccg gac agc ctg       853
gag gtg agc acg cgc tgg gcc ctg gac cgc gag cag cgg gag aag tac       901
gag ctg gtg gcc gtg tgc acc gtg cac gcc ggc gcg cgc gag gag gtg       949
gtg atg gtg ccc ttc ccg gtg acc gtg tac gac gag gac gac tcg gcg      997
ccc acc ttc ccc gcg ggc gtc gac acc gcc agc gcc gtg gtg gag ttc      1045
aag cgg aag gag gac acc gtg gtg gcc acg ctg cgt gtc ttc gat gca      1093
gac gtg gta cct gca tca ggg gag ctg gtg agg cgg tac aca agc acg      1141
ctg ctc ccc ggg gac acc tgg gcc cag cag acc ttc cgg gtg gaa cac      1189
tgg ccc aac gag acc tcg gtc cag gcc aac ggc agc ttc gtg cgg gcg      1237
acc gta cat gac tat agg ctg gtt ctc aac cgg aac ctc tcc atc tcg      1285
gag aac cgc acc atg cag ctg gcg gtg ctg gtc aat gac tca gac ttc      1333
cag ggc cca gga gcg ggc gtc ctc ttg ctc cac ttc aac gtg tcg gtg      1381
ctg ccg gtc agc ctg cac ctg ccc agt acc tac tcc ctc tcc gtg agc      1429
agg agg gct cgc cga ttt gcc cag atc ggg aaa gtc tgt gtg gaa aac      1477
tgc cag gca ttc agt ggc atc aac gtc cag tac aag ctg cat tcc tct      1525
ggt gcc aac tgc agc acg cta ggg gtg gtc acc tca gcc gag gac acc      1573
tcg ggg atc ctg ttt gtg aat gac acc aag gcc ctg cgg cgg ccc aag      1621
tgt gcc gaa ctt cac tac atg gtg gtg gcc acc gac cag cag acc tct      1669
agg cag gcc cag gcc cag ctg ctt gta aca gtg gag ggg tca tat gtg      1717
gcc gag gag gcg ggc tgc ccc ctg tcc tgt gca gtc agc aag aga cgg      1765
ctg gag tgt gag gag tgt ggc ggc ctg ggc tcc cca aca ggc agg tgt      1813
gag tgg agg caa gga gat ggc aaa ggg atc acc agg aac ttc tcc acc      1861
tgc tct ccc agc acc aag acc tgc ccc gac ggc cac tgc gat gtt gtg      1909
gag acc caa gac atc aac att tgc cct cag gac tgc ctc cgg ggc agc      1957
att gtt ggg gga cac gag cct ggg gag ccc cgg ggg att aaa gct ggc      2005
tat ggc acc tgc aac tgc ttc cct gag gag gag aag tgc ttc tgc gag      2053
ccc gaa gac atc cag gat cca ctg tgc gac gag ctg tgc cgc acg gtg      2101
atc gca gcc gct gtc ctc ttc tcc ttc atc gtc tcg gtg ctg ctg tct      2149
gcc ttc tgc atc cac tgc tac cac aag ttt gcc cac aag cca ccc atc      2197
tcc tca gct gag atg acc ttc cgg agg ccc gcc cag gcc ttc ccg gtc      2245
agc tac tcc tct tcc ggt gcc cgc cgg ccc tcg ctg gac tcc atg gag      2293
aac cag gtc tcc gtg gat gcc ttc aag atc ctg gag gat cca aag tgg      2341
gaa ttc cct cgg aag aac ttg gtt ctt gga aaa act cta gga gaa ggc      2389
gaa ttt gga aaa gtg gtc aag gca acg gcc ttc cat ctg aaa ggc aga      2437
gca ggg tac acc acg gtg gcc gtg aag atg ctg aaa gag aac gcc tcc      2485
ccg agt gag ctt cga gac ctg ctg tca gag ttc aac gtc ctg aag cag      2533
gtc aac cac cca cat gtc atc aaa ttg tat ggg gcc tgc agc cag gat      2581
ggc ccg ctc ctc ctc atc gtg gag tac gcc aaa tac ggc tcc ctg cgg      2629
ggc ttc ctc cgc gag agc cgc aaa gtg ggg cct ggc tac ctg ggc agt      2677
gga ggc agc cgc aac tcc agc tcc ctg gac cac ccg gat gag cgg gcc      2725
ctc acc atg ggc gac ctc atc tca ttt gcc tgg cag atc tca cag ggg      2773
atg cag tat ctg gcc gag atg aag ctc gtt cat cgg gac ttg gca gcc      2821
aga aac atc ctg gta gct gag ggg cgg aag atg aag att tcg gat ttc      2869
ggc ttg tcc cga gat gtt tat gaa gag gat tcc tac gtg aag agg agc      2917
cag ggt cgg att cca gtt aaa tgg atg gca att gaa tcc ctt ttt gat      2965
cat atc tac acc acg caa agt gat gta tgg tct ttt ggt gtc ctg ctg      3013
tgg gag atc gtg acc cta ggg gga aac ccc tat cct ggg att cct cct      3061
gag cgg ctc ttc aac ctt ctg aag acc ggc cac cgg atg gag agg cca      3109
gac aac tgc agc gag gag atg tac cgc ctg atg ctg caa tgc tgg aag      3157
cag gag ccg gac aaa agg ccg gtg ttt gcg gac  atc agc aaa gac ctg     3205
gag aag atg atg gtt  aag agg aga gac tac  ttg gac ctt gcg gcg        3250
tcc act cca tct gac  tcc ctg att tat gac  gac ggc ctc tca gag        3295
gag gag aca ccg ctg  gtg gac tgt aat aat  gcc ccc ctc cct cga        3340
gcc ctc cct tcc aca  tgg att gaa aac aaa  ctc tat ggc atg tca        3385
gac ccg aac tgg cct  gga gag agt cct gta  cca ctc acg aga gct        3430
gat ggc act aac act  ggg ttt cca aga tat  cca aat gat agt gta        3475
tat gct aac tgg atg  ctt tca ccc tca gcg  gca aaa tta atg gac        3520
acg ttt gat agt taa catttctttg tgaaaggtaa tggactcaca aggggaagaa      3575
acatgctgag aatggaaagt ctaccggccc tttctttgtg aacgtcacat tggccgagcc    3635
gtgttcagtt cccaggtggc agactcgttt ttggtagttt gttttaactt ccaaggtggt    3695
tttacttctg atagccggtg attttccctc ctagcagaca tgccacaccg ggtaagagct    3755
ctgagtctta gtggttaagc attcctttct cttcagtgcc cagcagcacc cagtgttggt    3815
ctgtgtccat cagtgaccac caacattctg tgttcacatg tgtgggtcca acacttacta    3875
cctggtgtat gaaattggac ctgaactgtt ggatttttct agttgccgcc aaacaaggca    3935
aaaaaattta aacatgaagc acacacacaa aaaaggcagt aggaaaaatg ctggccctga    3995
tgacctgtcc ttattcagaa tgagagactg cggggggggc ctgggggtag tgtcaatgcc     4055
cctccagggc tggaggggaa gaggggcccc gaggatgggc ctgggctcag cattcgagat    4115
cttgagaatg attttttttt aatcatgcaa cctttcctta ggaagacatt tggttttcat    4175
catgattaag atgattccta gatttagcac aatggagaga ttccatgcca tctttactat    4235
gtggatggtg gtatcaggga agagggctca caagacacat ttgtcccccg ggcccaccac    4295
atcatcctca cgtgttcggt actgagcagc cactacccct gatgagaaca gtatgaagaa    4355
agggggctgt tggagtccca gaattgctga cagcagaggc tttgctgctg tgaatcccac   4415
ctgccaccag cctgcagcac accccacagc caagtagagg cgaaagcagt ggctcatcct    4475
acctgttagg agcaggtagg gcttgtactc actttaattt gaatcttatc aacttactca    4535
taaagggaca ggctagctag ctgtgttaga agtagcaatg acaatgacca aggactgcta     4595
cacctctgat tacaattctg atgtgaaaaa gatggtgttt ggctcttata gagcctgtgt    4655
gaaaggccca tggatcagct cttcctgtgt ttgtaattta atgctgctac aagatgtttc    4715
tgtttcttag attctgacca tgactcataa gcttcttgtc attcttcatt gcttgtttgt    4775
ggtcacagat gcacaacact cctccagtct tgtgggggca gcttttggga agtctcagca    4835
gctcttctgg ctgtgttgtc agcactgtaa cttcgcagaa aagagtcgga ttaccaaaac    4895
actgcctgct cttcagactt aaagcactga taggacttaa aatagtctca ttcaaatact    4955
gtattttata taggcatttc acaaaaacag caaaattgtg gcattttgtg aggccaaggc    5015
ttggatgcgt gtgtaataga gccttgtggt gtgtgcgcac acacccagag ggagagtttg 5075
aaaaatgctt attggacacg taacctggct ctaatttggg ctgtttttca gatacactgt 5135
gataagttct tttacaaata tctatagaca tggtaaactt ttggttttca gatatgctta 5195
atgatagtct tactaaatgc agaaataaga ataaactttc tcaaattatt aaaaatgcct 5255
acacagtaag tgtgaattgc tgcaacaggt ttgttctcag gagggtaaga actccaggtc 5315
taaacagctg acccagtgat ggggaattta tccttgacca atttatcctt gaccaataac 5375
ctaattgtct attcctgagt tataaaagtc cccatcctta ttagctctac tggaattttc 5435
atacacgtaa atgcagaagt tactaagtat taagtattac tgagtattaa gtagtaatct 5495
gtcagttatt aaaatttgta aaatctattt atgaaaggtc attaaaccag atcatgttcc 5555
tttttttgta atcaaggtga ctaagaaaat cagttgtgta aataaaatca tgtatcataa 5615
aaaaaaaaaa aaaa 5629

<210> 4
<211> 1114
<212> PRT
<213> Homo sapiens
<400> 4

<210>  5
<211>  4174
<212>  DNA
<213>  Homo sapiens
<220>
<221>  CDS
<222>  (191)..(3409)
<223>
<400>  5
agtcccgcga ccgaagcagg gcgcgcagca gcgctgagtg ccccggaacg tgcgtcgcgc      60
ccccagtgtc cgtcgcgtcc gccgcgcccc gggcggggat ggggcggcca gactgagcgc      120
cgcacccgcc atccagaccc gccggcccta gccgcagtcc ctccagccgt ggccccagcg      180
cgcacgggcg atg gcg aag gcg acg tcc ggt gcc gcg ggg ctg cgt ctg      229
           Met Ala Lys Ala Thr Ser Gly Ala Ala Gly Leu Arg Leu
           1               5                   10
ctg ttg ctg ctg ctg ctg ccg ctg cta ggc aaa gtg gca ttg ggc ctc      277
Leu Leu Leu Leu Leu Leu Pro Leu Leu Gly Lys Val Ala Leu Gly Leu
    15                  20                  25
tac ttc tcg agg gat gct tac tgg gag aag ctg tat gtg gac cag gcg      325
Tyr Phe Ser Arg Asp Ala Tyr Trp Glu Lys Leu Tyr Val Asp Gln Ala
30                  35                  40                  45
gcc ggc acg ccc ttg ctg tac gtc cat gcc ctg cgg gac gcc cct gag      373
Ala Gly Thr Pro Leu Leu Tyr Val His Ala Leu Arg Asp Ala Pro Glu
                50                  55                  60
gag gtg ccc agc ttc cgc ctg ggc cag cat ctc tac ggc acg tac cgc      421
Glu Val Pro Ser Phe Arg Leu Gly Gln His Leu Tyr Gly Thr Tyr Arg
            65                  70                  75
aca cgg ctg cat gag aac aac tgg atc tgc atc cag gag gac acc ggc      469
Thr Arg Leu His Glu Asn Asn Trp Ile Cys Ile Gln Glu Asp Thr Gly
        80                  85                  90

ctc ctc tac ctt aac cgg agc ctg gac cat agc tcc tgg gag aag ctc 517
Leu Leu Tyr Leu Asn Arg Ser Leu Asp His Ser Ser Trp Glu Lys Leu
    95                  100                 105

agt gtc cgc aac cgc ggc ttt ccc ctg ctc acc gtc tac ctc aag gtc 565
Ser Val Arg Asn Arg Gly Phe Pro Leu Leu Thr Val Tyr Leu Lys Val
110                 115                 120                 125

ttc ctg tca ccc aca tcc ctt cgt gag ggc gag tgc cag tgg cca ggc 613
Phe Leu Ser Pro Thr Ser Leu Arg Glu Gly Glu Cys Gln Trp Pro Gly
                130                 135                 140

tgt gcc cgc gta tac ttc tcc ttc ttc aac acc tcc ttt cca gcc tgc 661
Cys Ala Arg Val Tyr Phe Ser Phe Phe Asn Thr Ser Phe Pro Ala Cys
            145                 150                 155

agc tcc ctc aag ccc cgg gag ctc tgc ttc cca gag aca agg ccc tcc 709
Ser Ser Leu Lys Pro Arg Glu Leu Cys Phe Pro Glu Thr Arg Pro Ser
        160                 165                 170

ttc cgc att cgg gag aac cga ccc cca ggc acc ttc cac cag ttc cgc 757
Phe Arg Ile Arg Glu Asn Arg Pro Pro Gly Thr Phe His Gln Phe Arg
    175                 180                 185

ctg ctg cct gtg cag ttc ttg tgc ccc aac atc agc gtg gcc tac agg 805
Leu Leu Pro Val Gln Phe Leu Cys Pro Asn Ile Ser Val Ala Tyr Arg
190                 195                 200                 205

ctc ctg gag ggt gag ggt ctg ccc ttc cgc tgc gcc ccg gac agc ctg 853
Leu Leu Glu Gly Glu Gly Leu Pro Phe Arg Cys Ala Pro Asp Ser Leu
                210                 215                 220

gag gtg agc acg cgc tgg gcc ctg gac cgc gag cag cgg gag aag tac 901
Glu Val Ser Thr Arg Trp Ala Leu Asp Arg Glu Gln Arg Glu Lys Tyr
            225                 230                 235

gag ctg gtg gcc gtg tgc acc gtg cac gcc ggc gcg cgc gag gag gtg 949
Glu Leu Val Ala Val Cys Thr Val His Ala Gly Ala Arg Glu Glu Val
        240                 245                 250

gtg atg gtg ccc ttc ccg gtg acc gtg tac gac gag gac gac tcg gcg 997
Val Met Val Pro Phe Pro Val Thr Val Tyr Asp Glu Asp Asp Ser Ala
    255                 260                 265

ccc acc ttc ccc gcg ggc gtc gac acc gcc agc gcc gtg gtg gag ttc 1045
Pro Thr Phe Pro Ala Gly Val Asp Thr Ala Ser Ala Val Val Glu Phe
270                 275                 280                 285

aag cgg aag gag gac acc gtg gtg gcc acg ctg cgt gtc ttc gat gca 1093
Lys Arg Lys Glu Asp Thr Val Val Ala Thr Leu Arg Val Phe Asp Ala
                290                 295                 300

gac gtg gta cct gca tca ggg gag ctg gtg agg cgg tac aca agc acg 1141
Asp Val Val Pro Ala Ser Gly Glu Leu Val Arg Arg Tyr Thr Ser Thr
            305                 310                 315

ctg ctc ccc ggg gac acc tgg gcc cag cag acc ttc cgg gtg gaa cac 1189
Leu Leu Pro Gly Asp Thr Trp Ala Gln Gln Thr Phe Arg Val Glu His
        320                 325                 330

tgg ccc aac gag acc tcg gtc cag gcc aac ggc agc ttc gtg cgg gcg 1237
Trp Pro Asn Glu Thr Ser Val Gln Ala Asn Gly Ser Phe Val Arg Ala
    335                 340                 345

acc gta cat gac tat agg ctg gtt ctc aac cgg aac ctc tcc atc tcg 1285
Thr Val His Asp Tyr Arg Leu Val Leu Asn Arg Asn Leu Ser Ile Ser
350                 355                 360                 365

gag aac cgc acc atg cag ctg gcg gtg ctg gtc aat gac tca gac ttc 1333
Glu Asn Arg Thr Met Gln Leu Ala Val Leu Val Asn Asp Ser Asp Phe
                370                 375                 380

cag ggc cca gga gcg ggc gtc ctc ttg ctc cac ttc aac gtg tcg gtg 1381
Gln Gly Pro Gly Ala Gly Val Leu Leu Leu His Phe Asn Val Ser Val
            385                 390                 395

ctg ccg gtc agc ctg cac ctg ccc agt acc tac tcc ctc tcc gtg agc 1429
Leu Pro Val Ser Leu His Leu Pro Ser Thr Tyr Ser Leu Ser Val Ser
        400                 405                 410

agg agg gct cgc cga ttt gcc cag atc ggg aaa gtc tgt gtg gaa aac 1477
Arg Arg Ala Arg Arg Phe Ala Gln Ile Gly Lys Val Cys Val Glu Asn
    415                 420                 425

tgc cag gca ttc agt ggc atc aac gtc cag tac aag ctg cat tcc tct 1525
Cys Gln Ala Phe Ser Gly Ile Asn Val Gln Tyr Lys Leu His Ser Ser
430                 435                 440                 445
ggt gcc aac tgc agc acg cta ggg gtg gtc acc tca gcc gag gac acc 1573
Gly Ala Asn Cys Ser Thr Leu Gly Val Val Thr Ser Ala Glu Asp Thr
                450                 455                 460
tcg ggg atc ctg ttt gtg aat gac acc aag gcc ctg cgg cgg ccc aag 1621
Ser Gly Ile Leu Phe Val Asn Asp Thr Lys Ala Leu Arg Arg Pro Lys
            465                 470                 475
tgt gcc gaa ctt cac tac atg gtg gtg gcc acc gac cag cag acc tct 1669
Cys Ala Glu Leu His Tyr Met Val Val Ala Thr Asp Gln Gln Thr Ser
        480                 485                 490
agg cag gcc cag gcc cag ctg ctt gta aca gtg gag ggg tca tat gtg 1717
Arg Gln Ala Gln Ala Gln Leu Leu Val Thr Val Glu Gly Ser Tyr Val
    495                 500                 505
gcc gag gag gcg ggc tgc ccc ctg tcc tgt gca gtc agc aag aga cgg 1765
Ala Glu Glu Ala Gly Cys Pro Leu Ser Cys Ala Val Ser Lys Arg Arg
510                 515                 520                 525
ctg gag tgt gag gag tgt ggc ggc ctg ggc tcc cca aca ggc agg tgt 1813
Leu Glu Cys Glu Glu Cys Gly Gly Leu Gly Ser Pro Thr Gly Arg Cys
                530                 535                 540
gag tgg agg caa gga gat ggc aaa ggg atc acc agg aac ttc tcc acc 1861
Glu Trp Arg Gln Gly Asp Gly Lys Gly Ile Thr Arg Asn Phe Ser Thr
            545                 550                 555
tgc tct ccc agc acc aag acc tgc ccc gac ggc cac tgc gat gtt gtg 1909
Cys Ser Pro Ser Thr Lys Thr Cys Pro Asp Gly His Cys Asp Val Val
        560                 565                 570
gag acc caa gac atc aac att tgc cct cag gac tgc ctc cgg ggc agc 1957
Glu Thr Gln Asp Ile Asn Ile Cys Pro Gln Asp Cys Leu Arg Gly Ser
    575                 580                 585
att gtt ggg gga cac gag cct ggg gag ccc cgg ggg att aaa gct ggc 2005
Ile Val Gly Gly His Glu Pro Gly Glu Pro Arg Gly Ile Lys Ala Gly
590                 595                 600                 605
tat ggc acc tgc aac tgc ttc cct gag gag gag aag tgc ttc tgc gag 2053
Tyr Gly Thr Cys Asn Cys Phe Pro Glu Glu Glu Lys Cys Phe Cys Glu
                610                 615                 620
ccc gaa gac atc cag gat cca ctg tgc gac gag ctg tgc cgc acg gtg 2101
Pro Glu Asp Ile Gln Asp Pro Leu Cys Asp Glu Leu Cys Arg Thr Val
            625                 630                 635
atc gca gcc gct gtc ctc ttc tcc ttc atc gtc tcg gtg ctg ctg tct 2149
Ile Ala Ala Ala Val Leu Phe Ser Phe Ile Val Ser Val Leu Leu Ser
        640                 645                 650
gcc ttc tgc atc cac tgc tac cac aag ttt gcc cac aag cca ccc atc 2197
Ala Phe Cys Ile His Cys Tyr His Lys Phe Ala His Lys Pro Pro Ile
    655                 660                 665
tcc tca gct gag atg acc ttc cgg agg ccc gcc cag gcc ttc ccg gtc 2245
Ser Ser Ala Glu Met Thr Phe Arg Arg Pro Ala Gln Ala Phe Pro Val
670                 675                 680                 685
agc tac tcc tct tcc ggt gcc cgc cgg ccc tcg ctg gac tcc atg gag 2293
Ser Tyr Ser Ser Ser Gly Ala Arg Arg Pro Ser Leu Asp Ser Met Glu
                690                 695                 700
aac cag gtc tcc gtg gat gcc ttc aag atc ctg gag gat cca aag tgg 2341
Asn Gln Val Ser Val Asp Ala Phe Lys Ile Leu Glu Asp Pro Lys Trp
            705                 710                 715
gaa ttc cct cgg aag aac ttg gtt ctt gga aaa act cta gga gaa ggc 2389
Glu Phe Pro Arg Lys Asn Leu Val Leu Gly Lys Thr Leu Gly Glu Gly
        720                 725                 730
gaa ttt gga aaa gtg gtc aag gca acg gcc ttc cat ctg aaa ggc aga 2437
Glu Phe Gly Lys Val Val Lys Ala Thr Ala Phe His Leu Lys Gly Arg
    735                 740                 745
gca ggg tac acc acg gtg gcc gtg aag atg ctg aaa gag aac gcc tcc 2485
Ala Gly Tyr Thr Thr Val Ala Val Lys Met Leu Lys Glu Asn Ala Ser
750                 755                 760                 765

ccg agt gag ctt cga gac ctg ctg tca gag ttc aac gtc ctg aag cag     2533
Pro Ser Glu Leu Arg Asp Leu Leu Ser Glu Phe Asn Val Leu Lys Gln
            770                 775                 780
gtc aac cac cca cat gtc atc aaa ttg tat ggg gcc tgc agc cag gat     2581
Val Asn His Pro His Val Ile Lys Leu Tyr Gly Ala Cys Ser Gln Asp
        785                 790                 795
ggc ccg ctc ctc ctc atc gtg gag tac gcc aaa tac ggc tcc ctg cgg     2629
Gly Pro Leu Leu Leu Ile Val Glu Tyr Ala Lys Tyr Gly Ser Leu Arg
    800                 805                 810
ggc ttc ctc cgc gag agc cgc aaa gtg ggg cct ggc tac ctg ggc agt     2677
Gly Phe Leu Arg Glu Ser Arg Lys Val Gly Pro Gly Tyr Leu Gly Ser
    815                 820                 825
gga ggc agc cgc aac tcc agc tcc ctg gac cac ccg gat gag cgg gcc     2725
Gly Gly Ser Arg Asn Ser Ser Ser Leu Asp His Pro Asp Glu Arg Ala
830                 835                 840                 845
ctc acc atg ggc gac ctc atc tca ttt gcc tgg cag atc tca cag ggg     2773
Leu Thr Met Gly Asp Leu Ile Ser Phe Ala Trp Gln Ile Ser Gln Gly
            850                 855                 860
atg cag tat ctg gcc gag atg aag ctc gtt cat cgg gac ttg gca gcc     2821
Met Gln Tyr Leu Ala Glu Met Lys Leu Val His Arg Asp Leu Ala Ala
        865                 870                 875
aga aac atc ctg gta gct gag ggg cgg aag atg aag att tcg gat ttc     2869
Arg Asn Ile Leu Val Ala Glu Gly Arg Lys Met Lys Ile Ser Asp Phe
    880                 885                 890
ggc ttg tcc cga gat gtt tat gaa gag gat tcc tac gtg aag agg agc     2917
Gly Leu Ser Arg Asp Val Tyr Glu Glu Asp Ser Tyr Val Lys Arg Ser
    895                 900                 905
cag ggt cgg att cca gtt aaa tgg atg gca att gaa tcc ctt ttt gat     2965
Gln Gly Arg Ile Pro Val Lys Trp Met Ala Ile Glu Ser Leu Phe Asp
910                 915                 920                 925
cat atc tac acc acg caa agt gat gta tgg tct ttt ggt gtc ctg ctg     3013
His Ile Tyr Thr Thr Gln Ser Asp Val Trp Ser Phe Gly Val Leu Leu
            930                 935                 940
tgg gag atc gtg acc cta ggg gga aac ccc tat cct ggg att cct cct     3061
Trp Glu Ile Val Thr Leu Gly Gly Asn Pro Tyr Pro Gly Ile Pro Pro
        945                 950                 955
gag cgg ctc ttc aac ctt ctg aag acc ggc cac cgg atg gag agg cca     3109
Glu Arg Leu Phe Asn Leu Leu Lys Thr Gly His Arg Met Glu Arg Pro
        960                 965                 970
gac aac tgc agc gag gag atg tac cgc ctg atg ctg caa tgc tgg aag     3157
Asp Asn Cys Ser Glu Glu Met Tyr Arg Leu Met Leu Gln Cys Trp Lys
    975                 980                 985
cag gag ccg gac aaa agg ccg gtg ttt gcg gac  atc agc aaa gac ctg     3205
Gln Glu Pro Asp Lys Arg Pro Val Phe Ala Asp  Ile Ser Lys Asp Leu
990                 995                 1000                1005
gag aag atg atg gtt  aag agg aga gac tac  ttg gac ctt gcg gcg     3250
Glu Lys Met Met Val  Lys Arg Arg Asp Tyr  Leu Asp Leu Ala Ala
            1010                1015                1020
tcc act cca tct gac  tcc ctg att tat gac  gac ggc ctc tca gag     3295
Ser Thr Pro Ser Asp  Ser Leu Ile Tyr Asp  Asp Gly Leu Ser Glu
            1025                1030                1035
gag gag aca ccg ctg  gtg gac tgt aat aat  gcc ccc ctc cct cga     3340
Glu Glu Thr Pro Leu  Val Asp Cys Asn Asn  Ala Pro Leu Pro Arg
            1040                1045                1050
gcc ctc cct tcc aca  tgg att gaa aac aaa  ctc tat ggt aga att     3385
Ala Leu Pro Ser Thr  Trp Ile Glu Asn Lys  Leu Tyr Gly Arg Ile
            1055                1060                1065
tcc cat gca ttt act  aga ttc tag caccgctgtc ccctctgcac tatccttcct     3439
Ser His Ala Phe Thr  Arg Phe
            1070
ctctgtgatg ctttttaaaa atgtttctgg tctgaacaaa accaaagtct gctctgaacc     3499
tttttatttg taaatgtctg actttgcatc cagtttacat ttaggcatta ttgcaactat     3559
gtttttctaa aaggaagtga aaataagtgt aattaccaca ttgcccagca acttaggatg     3619

gtagaggaaa aaacagatca gggcggaact ctcaggggag accaagaaca ggttgaataa 3679
ggcgcttctg gggtgggaat caagtcatag tacttctact ttaactaagt ggataaatat 3739
acaaatctgg ggaggtattc agttgagaaa ggagccacca gcaccactca gcctgcactg 3799
ggagcacagc caggttcccc cagacccctc ctgggcaggc aggtgcctct cagaggccac 3859
ccggcactgg cgagcagcca ctggccaagc ctcagcccca gtcccagcca catgtcctcc 3919
atcaggggta gcgaggttgc aggagctggc tggccctggg aggacgcacc cccactgctg 3979
ttttcacatc ctttccctta cccaccttca ggacggttgt cacttatgaa gtcagtgcta 4039
aagctggagc agttgctttt tgaaagaaca tggtctgtgg tgctgtggtc ttacaatgga 4099
cagtaaatat ggttcttgcc aaaactcctt cttttgtctt tgattaaata ctagaaattt 4159
aaaaaaaaaa aaaaa 4174

<210> 6
<211> 1072
<212> PRT
<213> Homo sapiens
<400> 6
Met Ala Lys Ala Thr Ser Gly Ala Ala Gly Leu Arg Leu Leu Leu Leu
1               5                   10                  15
Leu Leu Leu Pro Leu Leu Gly Lys Val Ala Leu Gly Leu Tyr Phe Ser
            20                  25                  30
Arg Asp Ala Tyr Trp Glu Lys Leu Tyr Val Asp Gln Ala Ala Gly Thr
        35                  40                  45
Pro Leu Leu Tyr Val His Ala Leu Arg Asp Ala Pro Glu Glu Val Pro
    50                  55                  60
Ser Phe Arg Leu Gly Gln His Leu Tyr Gly Thr Tyr Arg Thr Arg Leu
65                  70                  75                  80
His Glu Asn Asn Trp Ile Cys Ile Gln Glu Asp Thr Gly Leu Leu Tyr
                85                  90                  95
Leu Asn Arg Ser Leu Asp His Ser Ser Trp Glu Lys Leu Ser Val Arg
            100                 105                 110
Asn Arg Gly Phe Pro Leu Leu Thr Val Tyr Leu Lys Val Phe Leu Ser
        115                 120                 125
Pro Thr Ser Leu Arg Glu Gly Glu Cys Gln Trp Pro Gly Cys Ala Arg
    130                 135                 140
Val Tyr Phe Ser Phe Phe Asn Thr Ser Phe Pro Ala Cys Ser Ser Leu
145                 150                 155                 160
Lys Pro Arg Glu Leu Cys Phe Pro Glu Thr Arg Pro Ser Phe Arg Ile
                165                 170                 175
Arg Glu Asn Arg Pro Pro Gly Thr Phe His Gln Phe Arg Leu Leu Pro
            180                 185                 190
Val Gln Phe Leu Cys Pro Asn Ile Ser Val Ala Tyr Arg Leu Leu Glu
        195                 200                 205
Gly Glu Gly Leu Pro Phe Arg Cys Ala Pro Asp Ser Leu Glu Val Ser
    210                 215                 220
Thr Arg Trp Ala Leu Asp Arg Glu Gln Arg Glu Lys Tyr Glu Leu Val
225                 230                 235                 240
Ala Val Cys Thr Val His Ala Gly Ala Arg Glu Glu Val Val Met Val
                245                 250                 255
Pro Phe Pro Val Thr Val Tyr Asp Glu Asp Asp Ser Ala Pro Thr Phe
            260                 265                 270
Pro Ala Gly Val Asp Thr Ala Ser Ala Val Val Glu Phe Lys Arg Lys
        275                 280                 285
Glu Asp Thr Val Val Ala Thr Leu Arg Val Phe Asp Ala Asp Val Val
    290                 295                 300
Pro Ala Ser Gly Glu Leu Val Arg Arg Tyr Thr Ser Thr Leu Leu Pro
305                 310                 315                 320
Gly Asp Thr Trp Ala Gln Gln Thr Phe Arg Val Glu His Trp Pro Asn
                325                 330                 335
Glu Thr Ser Val Gln Ala Asn Gly Ser Phe Val Arg Ala Thr Val His
            340                 345                 350
Asp Tyr Arg Leu Val Leu Asn Arg Asn Leu Ser Ile Ser Glu Asn Arg
        355                 360                 365
Thr Met Gln Leu Ala Val Leu Val Asn Asp Ser Asp Phe Gln Gly Pro

370                 375                 380
Gly Ala Gly Val Leu Leu Leu His Phe Asn Val Ser Val Leu Pro Val
385                 390                 395                 400
Ser Leu His Leu Pro Ser Thr Tyr Ser Leu Ser Val Ser Arg Arg Ala
                405                 410                 415
Arg Arg Phe Ala Gln Ile Gly Lys Val Cys Val Glu Asn Cys Gln Ala
            420                 425                 430
Phe Ser Gly Ile Asn Val Gln Tyr Lys Leu His Ser Ser Gly Ala Asn
        435                 440                 445
Cys Ser Thr Leu Gly Val Val Thr Ser Ala Glu Asp Thr Ser Gly Ile
    450                 455                 460
Leu Phe Val Asn Asp Thr Lys Ala Leu Arg Arg Pro Lys Cys Ala Glu
465                 470                 475                 480
Leu His Tyr Met Val Val Ala Thr Asp Gln Gln Thr Ser Arg Gln Ala
                485                 490                 495
Gln Ala Gln Leu Leu Val Thr Val Glu Gly Ser Tyr Val Ala Glu Glu
            500                 505                 510
Ala Gly Cys Pro Leu Ser Cys Ala Val Ser Lys Arg Arg Leu Glu Cys
        515                 520                 525
Glu Glu Cys Gly Gly Leu Gly Ser Pro Thr Gly Arg Cys Glu Trp Arg
    530                 535                 540
Gln Gly Asp Gly Lys Gly Ile Thr Arg Asn Phe Ser Thr Cys Ser Pro
545                 550                 555                 560
Ser Thr Lys Thr Cys Pro Asp Gly His Cys Asp Val Val Glu Thr Gln
                565                 570                 575
Asp Ile Asn Ile Cys Pro Gln Asp Cys Leu Arg Gly Ser Ile Val Gly
            580                 585                 590
Gly His Glu Pro Gly Glu Pro Arg Gly Ile Lys Ala Gly Tyr Gly Thr
        595                 600                 605
Cys Asn Cys Phe Pro Glu Glu Glu Lys Cys Phe Cys Glu Pro Glu Asp
    610                 615                 620
Ile Gln Asp Pro Leu Cys Asp Glu Leu Cys Arg Thr Val Ile Ala Ala
625                 630                 635                 640
Ala Val Leu Phe Ser Phe Ile Val Ser Val Leu Leu Ser Ala Phe Cys
                645                 650                 655
Ile His Cys Tyr His Lys Phe Ala His Lys Pro Pro Ile Ser Ser Ala
            660                 665                 670
Glu Met Thr Phe Arg Arg Pro Ala Gln Ala Phe Pro Val Ser Tyr Ser
        675                 680                 685
Ser Ser Gly Ala Arg Arg Pro Ser Leu Asp Ser Met Glu Asn Gln Val
    690                 695                 700
Ser Val Asp Ala Phe Lys Ile Leu Glu Asp Pro Lys Trp Glu Phe Pro
705                 710                 715                 720
Arg Lys Asn Leu Val Leu Gly Lys Thr Leu Gly Glu Gly Glu Phe Gly
                725                 730                 735
Lys Val Val Lys Ala Thr Ala Phe His Leu Lys Gly Arg Ala Gly Tyr
            740                 745                 750
Thr Thr Val Ala Val Lys Met Leu Lys Glu Asn Ala Ser Pro Ser Glu
        755                 760                 765
Leu Arg Asp Leu Leu Ser Glu Phe Asn Val Leu Lys Gln Val Asn His
    770                 775                 780
Pro His Val Ile Lys Leu Tyr Gly Ala Cys Ser Gln Asp Gly Pro Leu
785                 790                 795                 800
Leu Leu Ile Val Glu Tyr Ala Lys Tyr Gly Ser Leu Arg Gly Phe Leu
                805                 810                 815
Arg Glu Ser Arg Lys Val Gly Pro Gly Tyr Leu Gly Ser Gly Gly Ser
            820                 825                 830
Arg Asn Ser Ser Ser Leu Asp His Pro Asp Glu Arg Ala Leu Thr Met
        835                 840                 845
Gly Asp Leu Ile Ser Phe Ala Trp Gln Ile Ser Gln Gly Met Gln Tyr
    850                 855                 860
Leu Ala Glu Met Lys Leu Val His Arg Asp Leu Ala Ala Arg Asn Ile
865                 870                 875                 880

Leu Val Ala Glu Gly Arg Lys Met Lys Ile Ser Asp Phe Gly Leu Ser
                885                 890                 895
Arg Asp Val Tyr Glu Glu Asp Ser Tyr Val Lys Arg Ser Gln Gly Arg
                900                 905                 910
Ile Pro Val Lys Trp Met Ala Ile Glu Ser Leu Phe Asp His Ile Tyr
        915                 920                 925
Thr Thr Gln Ser Asp Val Trp Ser Phe Gly Val Leu Leu Trp Glu Ile
    930                 935                 940
Val Thr Leu Gly Gly Asn Pro Tyr Pro Gly Ile Pro Pro Glu Arg Leu
945                 950                 955                 960
Phe Asn Leu Leu Lys Thr Gly His Arg Met Glu Arg Pro Asp Asn Cys
                965                 970                 975
Ser Glu Glu Met Tyr Arg Leu Met Leu Gln Cys Trp Lys Gln Glu Pro
                980                 985                 990
Asp Lys Arg Pro Val Phe Ala Asp  Ile Ser Lys Asp Leu  Glu Lys Met
        995                 1000                1005
Met Val  Lys Arg Arg Asp Tyr  Leu Asp Leu Ala Ala  Ser Thr Pro
    1010                1015                1020
Ser Asp  Ser Leu Ile Tyr Asp  Asp Gly Leu Ser Glu  Glu Glu Thr
    1025                1030                1035
Pro Leu  Val Asp Cys Asn Asn  Ala Pro Leu Pro Arg  Ala Leu Pro
    1040                1045                1050
Ser Thr  Trp Ile Glu Asn Lys  Leu Tyr Gly Arg Ile  Ser His Ala
    1055                1060                1065
Phe Thr  Arg Phe
    1070

<210>  7
<211>  4066
<212>  DNA
<213>  Homo sapiens
<220>
<221>  CDS
<222>  (305)..(1972)
<223>
<400>  7
cacacctgat ggtgtgactc ggccgacgcg agcgccgcgc ttcgcttcag ctgctagctg      60
gcccaaggga ggcgaccgcg gagggtggcg aggggcggcc aggacccgca gccccggggc     120
cgggccggtc cggaccgcca gggagggcag gtcagtgggc agatcgcgtc cgcgggattc     180
aatctctgcc cgctctgata acagtccttt tccctggcgc tcacttcgtg cctggcaccc     240
ggctgggcgc ctcaagaccg ttgtctcttc gatcgcttct ttggacttgg cgaccatttc     300
agag atg tct tcc aga agt acc aaa gat tta att aaa agt aag tgg gga      349
     Met Ser Ser Arg Ser Thr Lys Asp Leu Ile Lys Ser Lys Trp Gly
     1               5                   10                  15
tcg aag cct agt aac tcc aaa tcc gaa act aca tta gaa aaa tta aag      397
Ser Lys Pro Ser Asn Ser Lys Ser Glu Thr Thr Leu Glu Lys Leu Lys
                20                  25                  30
gga gaa att gca cac tta aag aca tca gtg gat gaa atc aca agt ggg      445
Gly Glu Ile Ala His Leu Lys Thr Ser Val Asp Glu Ile Thr Ser Gly
            35                  40                  45
aaa gga aag ctg act gat aaa gag aga cac aga ctt ttg gag aaa att      493
Lys Gly Lys Leu Thr Asp Lys Glu Arg His Arg Leu Leu Glu Lys Ile
        50                  55                  60
cga gtc ctt gag gct gag aag gag aag aat gct tat caa ctc aca gag      541
Arg Val Leu Glu Ala Glu Lys Glu Lys Asn Ala Tyr Gln Leu Thr Glu
    65                  70                  75
aag gac aaa gaa ata cag cga ctg aga gac caa ctg aag gcc aga tat      589
Lys Asp Lys Glu Ile Gln Arg Leu Arg Asp Gln Leu Lys Ala Arg Tyr
80                  85                  90                  95
agt act acc gca ttg ctt gaa cag ctg gaa gag aca acg aga gaa gga      637
Ser Thr Thr Ala Leu Leu Glu Gln Leu Glu Glu Thr Thr Arg Glu Gly
                100                 105                 110
gaa agg agg gag cag gtg ttg aaa gcc tta tct gaa gag aaa gac gta      685

Glu Arg Arg Glu Gln Val Leu Lys Ala Leu Ser Glu Glu Lys Asp Val
            115                 120                 125

ttg aaa caa cag ttg tct gct gca acc tca cga att gct gaa ctt gaa      733
Leu Lys Gln Gln Leu Ser Ala Ala Thr Ser Arg Ile Ala Glu Leu Glu
        130                 135                 140

agc aaa acc aat aca ctc cgt tta tca cag gag gat cca aag tgg gaa      781
Ser Lys Thr Asn Thr Leu Arg Leu Ser Gln Glu Asp Pro Lys Trp Glu
    145                 150                 155

ttc cct cgg aag aac ttg gtt ctt gga aaa act cta gga gaa ggc gaa      829
Phe Pro Arg Lys Asn Leu Val Leu Gly Lys Thr Leu Gly Glu Gly Glu
160                 165                 170                 175

ttt gga aaa gtg gtc aag gca acg gcc ttc cat ctg aaa ggc aga gca      877
Phe Gly Lys Val Val Lys Ala Thr Ala Phe His Leu Lys Gly Arg Ala
                180                 185                 190

ggg tac acc acg gtg gcc gtg aag atg ctg aaa gag aac gcc tcc ccg      925
Gly Tyr Thr Thr Val Ala Val Lys Met Leu Lys Glu Asn Ala Ser Pro
            195                 200                 205

agt gag ctt cga gac ctg ctg tca gag ttc aac gtc ctg aag cag gtc      973
Ser Glu Leu Arg Asp Leu Leu Ser Glu Phe Asn Val Leu Lys Gln Val
        210                 215                 220

aac cac cca cat gtc atc aaa ttg tat ggg gcc tgc agc cag gat ggc     1021
Asn His Pro His Val Ile Lys Leu Tyr Gly Ala Cys Ser Gln Asp Gly
    225                 230                 235

ccg ctc ctc ctc atc gtg gag tac gcc aaa tac ggc tcc ctg cgg ggc     1069
Pro Leu Leu Leu Ile Val Glu Tyr Ala Lys Tyr Gly Ser Leu Arg Gly
240                 245                 250                 255

ttc ctc cgc gag agc cgc aaa gtg ggg cct ggc tac ctg ggc agt gga     1117
Phe Leu Arg Glu Ser Arg Lys Val Gly Pro Gly Tyr Leu Gly Ser Gly
                260                 265                 270

ggc agc cgc aac tcc agc tcc ctg gac cac ccg gat gag cgg gcc ctc     1165
Gly Ser Arg Asn Ser Ser Ser Leu Asp His Pro Asp Glu Arg Ala Leu
            275                 280                 285

acc atg ggc gac ctc atc tca ttt gcc tgg cag atc tca cag ggg atg     1213
Thr Met Gly Asp Leu Ile Ser Phe Ala Trp Gln Ile Ser Gln Gly Met
        290                 295                 300

cag tat ctg gcc gag atg aag ctc gtt cat cgg gac ttg gca gcc aga     1261
Gln Tyr Leu Ala Glu Met Lys Leu Val His Arg Asp Leu Ala Ala Arg
    305                 310                 315

aac atc ctg gta gct gag ggg cgg aag atg aag att tcg gat ttc ggc     1309
Asn Ile Leu Val Ala Glu Gly Arg Lys Met Lys Ile Ser Asp Phe Gly
320                 325                 330                 335

ttg tcc cga gat gtt tat gaa gag gat tcc tac gtg aag agg agc cag     1357
Leu Ser Arg Asp Val Tyr Glu Glu Asp Ser Tyr Val Lys Arg Ser Gln
                340                 345                 350

ggt cgg att cca gtt aaa tgg atg gca att gaa tcc ctt ttt gat cat     1405
Gly Arg Ile Pro Val Lys Trp Met Ala Ile Glu Ser Leu Phe Asp His
            355                 360                 365

atc tac acc acg caa agt gat gta tgg tct ttt ggt gtc ctg ctg tgg     1453
Ile Tyr Thr Thr Gln Ser Asp Val Trp Ser Phe Gly Val Leu Leu Trp
        370                 375                 380

gag atc gtg acc cta ggg gga aac ccc tat cct ggg att cct cct gag     1501
Glu Ile Val Thr Leu Gly Gly Asn Pro Tyr Pro Gly Ile Pro Pro Glu
    385                 390                 395

cgg ctc ttc aac ctt ctg aag acc ggc cac cgg atg gag agg cca gac     1549
Arg Leu Phe Asn Leu Leu Lys Thr Gly His Arg Met Glu Arg Pro Asp
400                 405                 410                 415

aac tgc agc gag gag atg tac cgc ctg atg ctg caa tgc tgg aag cag     1597
Asn Cys Ser Glu Glu Met Tyr Arg Leu Met Leu Gln Cys Trp Lys Gln
                420                 425                 430

gag ccg gac aaa agg ccg gtg ttt gcg gac atc agc aaa gac ctg gag     1645
Glu Pro Asp Lys Arg Pro Val Phe Ala Asp Ile Ser Lys Asp Leu Glu
            435                 440                 445

aag atg atg gtt aag agg aga gac tac ttg gac ctt gcg gcg tcc act     1693

Lys Met Met Val Lys Arg Arg Asp Tyr Leu Asp Leu Ala Ala Ser Thr
        450                 455                 460
cca tct gac tcc ctg att tat gac gac ggc ctc tca gag gag gag aca      1741
Pro Ser Asp Ser Leu Ile Tyr Asp Asp Gly Leu Ser Glu Glu Glu Thr
    465                 470                 475
ccg ctg gtg gac tgt aat aat gcc ccc ctc cct cga gcc ctc cct tcc      1789
Pro Leu Val Asp Cys Asn Asn Ala Pro Leu Pro Arg Ala Leu Pro Ser
480                 485                 490                 495
aca tgg att gaa aac aaa ctc tat ggc atg tca gac ccg aac tgg cct      1837
Thr Trp Ile Glu Asn Lys Leu Tyr Gly Met Ser Asp Pro Asn Trp Pro
                500                 505                 510
gga gag agt cct gta cca ctc acg aga gct gat ggc act aac act ggg      1885
Gly Glu Ser Pro Val Pro Leu Thr Arg Ala Asp Gly Thr Asn Thr Gly
            515                 520                 525
ttt cca aga tat cca aat gat agt gta tat gct aac tgg atg ctt tca      1933
Phe Pro Arg Tyr Pro Asn Asp Ser Val Tyr Ala Asn Trp Met Leu Ser
        530                 535                 540
ccc tca gcg gca aaa tta atg gac acg ttt gat agt taa catttctttg      1982
Pro Ser Ala Ala Lys Leu Met Asp Thr Phe Asp Ser
    545                 550                 555
tgaaaggtaa tggactcaca aggggaagaa acatgctgag aatggaaagt ctaccggccc   2042
tttctttgtg aacgtcacat tggccgagcc gtgttcagtt cccaggtggc agactcgttt   2102
ttggtagttt gttttaactt ccaaggtggt tttacttctg atagccggtg attttccctc   2162
ctagcagaca tgccacaccg ggtaagagct ctgagtctta gtggttaagc attcctttct   2222
cttcagtgcc cagcagcacc cagtgttggt ctgtgtccat cagtgaccac caacattctg   2282
tgttcacatg tgtgggtcca acacttacta cctggtgtat gaaattggac ctgaactgtt   2342
ggatttttct agttgccgcc aaacaaggca aaaaaattta aacatgaagc acacacacaa   2402
aaaaggcagt aggaaaaatg ctggccctga tgacctgtcc ttattcagaa tgagagactg   2462
cggggggggc ctgggggtag tgtcaatgcc cctccagggc tggaggggaa gaggggcccc   2522
gaggatgggc ctgggctcag cattcgagat cttgagaatg attttttttt aatcatgcaa   2582
cctttcctta ggaagacatt tggttttcat catgattaag atgattccta gatttagcac   2642
aatggagaga ttccatgcca tctttactat gtggatggtg gtatcaggga agagggctca   2702
caagacacat ttgtcccccg ggcccaccac atcatcctca cgtgttcggt actgagcagc   2762
cactacccct gatgagaaca gtatgaagaa agggggctgt tggagtccca gaattgctga   2822
cagcagaggc tttgctgctg tgaatcccac ctgccaccag cctgcagcac accccacagc   2882
caagtagagg cgaaagcagt ggctcatcct acctgttagg agcaggtagg gcttgtactc   2942
actttaattt gaatcttatc aacttactca taaagggaca ggctagctag ctgtgttaga   3002
agtagcaatg acaatgacca aggactgcta cacctctgat tacaattctg atgtgaaaaa   3062
gatggtgttt ggctcttata gagcctgtgt gaaaggccca tggatcagct cttcctgtgt   3122
ttgtaattta atgctgctac aagatgtttc tgtttcttag attctgacca tgactcataa   3182
gcttcttgtc attcttcatt gcttgtttgt ggtcacagat gcacaacact cctccagtct   3242
tgtgggggca gcttttggga agtctcagca gctcttctgg ctgtgttgtc agcactgtaa   3302
cttcgcagaa aagagtcgga ttaccaaaac actgcctgct cttcagactt aaagcactga   3362
taggacttaa aatagtctca ttcaaatact gtattttata taggcatttc acaaaaacag   3422
caaaattgtg gcattttgtg aggccaaggc ttggatgcgt gtgtaataga gccttgtggt   3482
gtgtgcgcac acacccagag ggagagtttg aaaaatgctt attggacacg taacctggct   3542
ctaatttggg ctgtttttca gatacactgt gataagttct tttacaaata tctatagaca   3602
tggtaaactt ttggttttca gatatgctta atgatagtct tactaaatgc agaaataaga   3662
ataaactttc tcaaattatt aaaaatgcct acacagtaag tgtgaattgc tgcaacaggt   3722
ttgttctcag gagggtaaga actccaggtc taaacagctg acccagtgat ggggaattta   3782
tccttgacca atttatcctt gaccaataac ctaattgtct attcctgagt tataaaagtc   3842
cccatcctta ttagctctac tggaattttc atacacgtaa atgcagaagt tactaagtat   3902
taagtattac tgagtattaa gtagtaatct gtcagttatt aaaatttgta aaatctattt   3962
atgaaaggtc attaaaccag atcatgttcc tttttttgta atcaaggtga ctaagaaaat   4022
cagttgtgta aataaaatca tgtatcataa aaaaaaaaaa aaaa                    4066

<210>  8
<211>  555
<212>  PRT
<213>  Homo sapiens
<400>  8
Met Ser Ser Arg Ser Thr Lys Asp Leu Ile Lys Ser Lys Trp Gly Ser
1               5                   10                  15

Lys Pro Ser Asn Ser Lys Ser Glu Thr Thr Leu Glu Lys Leu Lys Gly
            20                  25                  30
Glu Ile Ala His Leu Lys Thr Ser Val Asp Glu Ile Thr Ser Gly Lys
        35                  40                  45
Gly Lys Leu Thr Asp Lys Glu Arg His Arg Leu Leu Glu Lys Ile Arg
    50                  55                  60
Val Leu Glu Ala Glu Lys Glu Lys Asn Ala Tyr Gln Leu Thr Glu Lys
65                  70                  75                  80
Asp Lys Glu Ile Gln Arg Leu Arg Asp Gln Leu Lys Ala Arg Tyr Ser
                85                  90                  95
Thr Thr Ala Leu Leu Glu Gln Leu Glu Glu Thr Thr Arg Glu Gly Glu
            100                 105                 110
Arg Arg Glu Gln Val Leu Lys Ala Leu Ser Glu Glu Lys Asp Val Leu
        115                 120                 125
Lys Gln Gln Leu Ser Ala Ala Thr Ser Arg Ile Ala Glu Leu Glu Ser
    130                 135                 140
Lys Thr Asn Thr Leu Arg Leu Ser Gln Glu Asp Pro Lys Trp Glu Phe
145                 150                 155                 160
Pro Arg Lys Asn Leu Val Leu Gly Lys Thr Leu Gly Glu Gly Glu Phe
                165                 170                 175
Gly Lys Val Val Lys Ala Thr Ala Phe His Leu Lys Gly Arg Ala Gly
            180                 185                 190
Tyr Thr Thr Val Ala Val Lys Met Leu Lys Glu Asn Ala Ser Pro Ser
        195                 200                 205
Glu Leu Arg Asp Leu Leu Ser Glu Phe Asn Val Leu Lys Gln Val Asn
    210                 215                 220
His Pro His Val Ile Lys Leu Tyr Gly Ala Cys Ser Gln Asp Gly Pro
225                 230                 235                 240
Leu Leu Leu Ile Val Glu Tyr Ala Lys Tyr Gly Ser Leu Arg Gly Phe
                245                 250                 255
Leu Arg Glu Ser Arg Lys Val Gly Pro Gly Tyr Leu Gly Ser Gly Gly
            260                 265                 270
Ser Arg Asn Ser Ser Ser Leu Asp His Pro Asp Glu Arg Ala Leu Thr
        275                 280                 285
Met Gly Asp Leu Ile Ser Phe Ala Trp Gln Ile Ser Gln Gly Met Gln
    290                 295                 300
Tyr Leu Ala Glu Met Lys Leu Val His Arg Asp Leu Ala Ala Arg Asn
305                 310                 315                 320
Ile Leu Val Ala Glu Gly Arg Lys Met Lys Ile Ser Asp Phe Gly Leu
                325                 330                 335
Ser Arg Asp Val Tyr Glu Glu Asp Ser Tyr Val Lys Arg Ser Gln Gly
            340                 345                 350
Arg Ile Pro Val Lys Trp Met Ala Ile Glu Ser Leu Phe Asp His Ile
        355                 360                 365
Tyr Thr Thr Gln Ser Asp Val Trp Ser Phe Gly Val Leu Leu Trp Glu
    370                 375                 380
Ile Val Thr Leu Gly Gly Asn Pro Tyr Pro Gly Ile Pro Pro Glu Arg
385                 390                 395                 400
Leu Phe Asn Leu Leu Lys Thr Gly His Arg Met Glu Arg Pro Asp Asn
                405                 410                 415
Cys Ser Glu Glu Met Tyr Arg Leu Met Leu Gln Cys Trp Lys Gln Glu
            420                 425                 430
Pro Asp Lys Arg Pro Val Phe Ala Asp Ile Ser Lys Asp Leu Glu Lys
        435                 440                 445
Met Met Val Lys Arg Arg Asp Tyr Leu Asp Leu Ala Ala Ser Thr Pro
    450                 455                 460
Ser Asp Ser Leu Ile Tyr Asp Asp Gly Leu Ser Glu Glu Glu Thr Pro
465                 470                 475                 480
Leu Val Asp Cys Asn Asn Ala Pro Leu Pro Arg Ala Leu Pro Ser Thr
                485                 490                 495
Trp Ile Glu Asn Lys Leu Tyr Gly Met Ser Asp Pro Asn Trp Pro Gly
            500                 505                 510
Glu Ser Pro Val Pro Leu Thr Arg Ala Asp Gly Thr Asn Thr Gly Phe

515                 520                 525
Pro Arg Tyr Pro Asn Asp Ser Val Tyr Ala Asn Trp Met Leu Ser Pro
    530                 535                 540
Ser Ala Ala Lys Leu Met Asp Thr Phe Asp Ser
545                 550                 555

<210>  9
<211>  2611
<212>  DNA
<213>  Homo sapiens
<220>
<221>  CDS
<222>  (305)..(1846)
<223>
<400>  9
cacacctgat ggtgtgactc ggccgacgcg agcgccgcgc ttcgcttcag ctgctagctg      60
gcccaaggga ggcgaccgcg gagggtggcg aggggcggcc aggacccgca gccccggggc     120
cgggccggtc cggaccgcca gggagggcag gtcagtgggc agatcgcgtc cgcgggattc     180
aatctctgcc cgctctgata acagtccttt tccctggcgc tcacttcgtg cctggcaccc     240
ggctgggcgc ctcaagaccg ttgtctcttc gatcgcttct ttggacttgg cgaccatttc     300
agag atg tct tcc aga agt acc aaa gat tta att aaa agt aag tgg gga      349
     Met Ser Ser Arg Ser Thr Lys Asp Leu Ile Lys Ser Lys Trp Gly
     1               5                   10                  15
tcg aag cct agt aac tcc aaa tcc gaa act aca tta gaa aaa tta aag      397
Ser Lys Pro Ser Asn Ser Lys Ser Glu Thr Thr Leu Glu Lys Leu Lys
                20                  25                  30
gga gaa att gca cac tta aag aca tca gtg gat gaa atc aca agt ggg      445
Gly Glu Ile Ala His Leu Lys Thr Ser Val Asp Glu Ile Thr Ser Gly
            35                  40                  45
aaa gga aag ctg act gat aaa gag aga cac aga ctt ttg gag aaa att      493
Lys Gly Lys Leu Thr Asp Lys Glu Arg His Arg Leu Leu Glu Lys Ile
        50                  55                  60
cga gtc ctt gag gct gag aag gag aag aat gct tat caa ctc aca gag      541
Arg Val Leu Glu Ala Glu Lys Glu Lys Asn Ala Tyr Gln Leu Thr Glu
    65                  70                  75
aag gac aaa gaa ata cag cga ctg aga gac caa ctg aag gcc aga tat      589
Lys Asp Lys Glu Ile Gln Arg Leu Arg Asp Gln Leu Lys Ala Arg Tyr
80                  85                  90                  95
agt act acc gca ttg ctt gaa cag ctg gaa gag aca acg aga gaa gga      637
Ser Thr Thr Ala Leu Leu Glu Gln Leu Glu Glu Thr Thr Arg Glu Gly
                100                 105                 110
gaa agg agg gag cag gtg ttg aaa gcc tta tct gaa gag aaa gac gta      685
Glu Arg Arg Glu Gln Val Leu Lys Ala Leu Ser Glu Glu Lys Asp Val
            115                 120                 125
ttg aaa caa cag ttg tct gct gca acc tca cga att gct gaa ctt gaa      733
Leu Lys Gln Gln Leu Ser Ala Ala Thr Ser Arg Ile Ala Glu Leu Glu
        130                 135                 140
agc aaa acc aat aca ctc cgt tta tca cag gag gat cca aag tgg gaa      781
Ser Lys Thr Asn Thr Leu Arg Leu Ser Gln Glu Asp Pro Lys Trp Glu
    145                 150                 155
ttc cct cgg aag aac ttg gtt ctt gga aaa act cta gga gaa ggc gaa      829
Phe Pro Arg Lys Asn Leu Val Leu Gly Lys Thr Leu Gly Glu Gly Glu
160                 165                 170                 175
ttt gga aaa gtg gtc aag gca acg gcc ttc cat ctg aaa ggc aga gca      877
Phe Gly Lys Val Val Lys Ala Thr Ala Phe His Leu Lys Gly Arg Ala
                180                 185                 190
ggg tac acc acg gtg gcc gtg aag atg ctg aaa gag aac gcc tcc ccg      925
Gly Tyr Thr Thr Val Ala Val Lys Met Leu Lys Glu Asn Ala Ser Pro
            195                 200                 205
agt gag ctt cga gac ctg ctg tca gag ttc aac gtc ctg aag cag gtc      973
Ser Glu Leu Arg Asp Leu Leu Ser Glu Phe Asn Val Leu Lys Gln Val
        210                 215                 220
aac cac cca cat gtc atc aaa ttg tat ggg gcc tgc agc cag gat ggc     1021

Asn His Pro His Val Ile Lys Leu Tyr Gly Ala Cys Ser Gln Asp Gly
    225                 230                 235
ccg ctc ctc ctc atc gtg gag tac gcc aaa tac ggc tcc ctg cgg ggc      1069
Pro Leu Leu Leu Ile Val Glu Tyr Ala Lys Tyr Gly Ser Leu Arg Gly
240                 245                 250                 255
ttc ctc cgc gag agc cgc aaa gtg ggg cct ggc tac ctg ggc agt gga      1117
Phe Leu Arg Glu Ser Arg Lys Val Gly Pro Gly Tyr Leu Gly Ser Gly
                260                 265                 270
ggc agc cgc aac tcc agc tcc ctg gac cac ccg gat gag cgg gcc ctc      1165
Gly Ser Arg Asn Ser Ser Ser Leu Asp His Pro Asp Glu Arg Ala Leu
            275                 280                 285
acc atg ggc gac ctc atc tca ttt gcc tgg cag atc tca cag ggg atg      1213
Thr Met Gly Asp Leu Ile Ser Phe Ala Trp Gln Ile Ser Gln Gly Met
        290                 295                 300
cag tat ctg gcc gag atg aag ctc gtt cat cgg gac ttg gca gcc aga      1261
Gln Tyr Leu Ala Glu Met Lys Leu Val His Arg Asp Leu Ala Ala Arg
    305                 310                 315
aac atc ctg gta gct gag ggg cgg aag atg aag att tcg gat ttc ggc      1309
Asn Ile Leu Val Ala Glu Gly Arg Lys Met Lys Ile Ser Asp Phe Gly
320                 325                 330                 335
ttg tcc cga gat gtt tat gaa gag gat tcc tac gtg aag agg agc cag      1357
Leu Ser Arg Asp Val Tyr Glu Glu Asp Ser Tyr Val Lys Arg Ser Gln
                340                 345                 350
ggt cgg att cca gtt aaa tgg atg gca att gaa tcc ctt ttt gat cat      1405
Gly Arg Ile Pro Val Lys Trp Met Ala Ile Glu Ser Leu Phe Asp His
            355                 360                 365
atc tac acc acg caa agt gat gta tgg tct ttt ggt gtc ctg ctg tgg      1453
Ile Tyr Thr Thr Gln Ser Asp Val Trp Ser Phe Gly Val Leu Leu Trp
        370                 375                 380
gag atc gtg acc cta ggg gga aac ccc tat cct ggg att cct cct gag      1501
Glu Ile Val Thr Leu Gly Gly Asn Pro Tyr Pro Gly Ile Pro Pro Glu
    385                 390                 395
cgg ctc ttc aac ctt ctg aag acc ggc cac cgg atg gag agg cca gac      1549
Arg Leu Phe Asn Leu Leu Lys Thr Gly His Arg Met Glu Arg Pro Asp
400                 405                 410                 415
aac tgc agc gag gag atg tac cgc ctg atg ctg caa tgc tgg aag cag      1597
Asn Cys Ser Glu Glu Met Tyr Arg Leu Met Leu Gln Cys Trp Lys Gln
                420                 425                 430
gag ccg gac aaa agg ccg gtg ttt gcg gac atc agc aaa gac ctg gag      1645
Glu Pro Asp Lys Arg Pro Val Phe Ala Asp Ile Ser Lys Asp Leu Glu
            435                 440                 445
aag atg atg gtt aag agg aga gac tac ttg gac ctt gcg gcg tcc act      1693
Lys Met Met Val Lys Arg Arg Asp Tyr Leu Asp Leu Ala Ala Ser Thr
        450                 455                 460
cca tct gac tcc ctg att tat gac gac ggc ctc tca gag gag gag aca      1741
Pro Ser Asp Ser Leu Ile Tyr Asp Asp Gly Leu Ser Glu Glu Glu Thr
    465                 470                 475
ccg ctg gtg gac tgt aat aat gcc ccc ctc cct cga gcc ctc cct tcc      1789
Pro Leu Val Asp Cys Asn Asn Ala Pro Leu Pro Arg Ala Leu Pro Ser
480                 485                 490                 495
aca tgg att gaa aac aaa ctc tat ggt aga att tcc cat gca ttt act      1837
Thr Trp Ile Glu Asn Lys Leu Tyr Gly Arg Ile Ser His Ala Phe Thr
                500                 505                 510
aga ttc tag caccgctgtc ccctctgcac tatccttcct ctctgtgatg              1886
Arg Phe
ctttttaaaa atgtttctgg tctgaacaaa accaaagtct gctctgaacc tttttatttg    1946
taaatgtctg actttgcatc cagtttacat ttaggcatta ttgcaactat gtttttctaa    2006
aaggaagtga aaataagtgt aattaccaca ttgcccagca acttaggatg gtagaggaaa    2066
aaacagatca gggcggaact ctcaggggag accaagaaca ggttgaataa ggcgcttctg    2126
gggtgggaat caagtcatag tacttctact ttaactaagt ggataaatat acaaatctgg    2186
ggaggtattc agttgagaaa ggagccacca gcaccactca gcctgcactg ggagcacagc    2246
caggttcccc cagacccctc ctgggcaggc aggtgcctct cagaggccac ccggcactgg    2306
cgagcagcca ctggccaagc ctcagcccca gtcccagcca catgtcctcc atcaggggta    2366

gcgaggttgc aggagctggc tggccctggg aggacgcacc cccactgctg ttttcacatc 2426
ctttccctta cccaccttca ggacggttgt cacttatgaa gtcagtgcta aagctggagc 2486
agttgctttt tgaaagaaca tggtctgtgg tgctgtggtc ttacaatgga cagtaaatat 2546
ggttcttgcc aaaactcctt cttttgtctt tgattaaata ctagaaattt aaaaaaaaaa 2606
aaaaa 2611

<210> 10
<211> 513
<212> PRT
<213> Homo sapiens
<400> 10
Met Ser Ser Arg Ser Thr Lys Asp Leu Ile Lys Ser Lys Trp Gly Ser
1               5                   10                  15
Lys Pro Ser Asn Ser Lys Ser Glu Thr Thr Leu Glu Lys Leu Lys Gly
            20                  25                  30
Glu Ile Ala His Leu Lys Thr Ser Val Asp Glu Ile Thr Ser Gly Lys
        35                  40                  45
Gly Lys Leu Thr Asp Lys Glu Arg His Arg Leu Leu Glu Lys Ile Arg
    50                  55                  60
Val Leu Glu Ala Glu Lys Glu Lys Asn Ala Tyr Gln Leu Thr Glu Lys
65                  70                  75                  80
Asp Lys Glu Ile Gln Arg Leu Arg Asp Gln Leu Lys Ala Arg Tyr Ser
                85                  90                  95
Thr Thr Ala Leu Leu Glu Gln Leu Glu Glu Thr Thr Arg Glu Gly Glu
            100                 105                 110
Arg Arg Glu Gln Val Leu Lys Ala Leu Ser Glu Glu Lys Asp Val Leu
        115                 120                 125
Lys Gln Gln Leu Ser Ala Ala Thr Ser Arg Ile Ala Glu Leu Glu Ser
    130                 135                 140
Lys Thr Asn Thr Leu Arg Leu Ser Gln Glu Asp Pro Lys Trp Glu Phe
145                 150                 155                 160
Pro Arg Lys Asn Leu Val Leu Gly Lys Thr Leu Gly Glu Gly Glu Phe
                165                 170                 175
Gly Lys Val Val Lys Ala Thr Ala Phe His Leu Lys Gly Arg Ala Gly
            180                 185                 190
Tyr Thr Thr Val Ala Val Lys Met Leu Lys Glu Asn Ala Ser Pro Ser
        195                 200                 205
Glu Leu Arg Asp Leu Leu Ser Glu Phe Asn Val Leu Lys Gln Val Asn
    210                 215                 220
His Pro His Val Ile Lys Leu Tyr Gly Ala Cys Ser Gln Asp Gly Pro
225                 230                 235                 240
Leu Leu Leu Ile Val Glu Tyr Ala Lys Tyr Gly Ser Leu Arg Gly Phe
                245                 250                 255
Leu Arg Glu Ser Arg Lys Val Gly Pro Gly Tyr Leu Gly Ser Gly Gly
            260                 265                 270
Ser Arg Asn Ser Ser Ser Leu Asp His Pro Asp Glu Arg Ala Leu Thr
        275                 280                 285
Met Gly Asp Leu Ile Ser Phe Ala Trp Gln Ile Ser Gln Gly Met Gln
    290                 295                 300
Tyr Leu Ala Glu Met Lys Leu Val His Arg Asp Leu Ala Ala Arg Asn
305                 310                 315                 320
Ile Leu Val Ala Glu Gly Arg Lys Met Lys Ile Ser Asp Phe Gly Leu
                325                 330                 335
Ser Arg Asp Val Tyr Glu Glu Asp Ser Tyr Val Lys Arg Ser Gln Gly
            340                 345                 350
Arg Ile Pro Val Lys Trp Met Ala Ile Glu Ser Leu Phe Asp His Ile
        355                 360                 365
Tyr Thr Thr Gln Ser Asp Val Trp Ser Phe Gly Val Leu Leu Trp Glu
    370                 375                 380
Ile Val Thr Leu Gly Gly Asn Pro Tyr Pro Gly Ile Pro Pro Glu Arg
385                 390                 395                 400
Leu Phe Asn Leu Leu Lys Thr Gly His Arg Met Glu Arg Pro Asp Asn
                405                 410                 415

Cys Ser Glu Glu Met Tyr Arg Leu Met Leu Gln Cys Trp Lys Gln Glu
            420                 425                 430
Pro Asp Lys Arg Pro Val Phe Ala Asp Ile Ser Lys Asp Leu Glu Lys
        435                 440                 445
Met Met Val Lys Arg Arg Asp Tyr Leu Asp Leu Ala Ala Ser Thr Pro
    450                 455                 460
Ser Asp Ser Leu Ile Tyr Asp Asp Gly Leu Ser Glu Glu Glu Thr Pro
465                 470                 475                 480
Leu Val Asp Cys Asn Asn Ala Pro Leu Pro Arg Ala Leu Pro Ser Thr
                485                 490                 495
Trp Ile Glu Asn Lys Leu Tyr Gly Arg Ile Ser His Ala Phe Thr Arg
            500                 505                 510
Phe

## Description

### TECHNICAL FIELD

The present invention relates to fusion genes between the CEP55 gene and the RET gene, and more particularly to polynucleotides encoding fusion polypeptides between CEP55 protein or part thereof and RET protein or part thereof, polypeptides encoded by said polynucleotides, and a method for detecting said polynucleotides or polypeptides. This invention also relates to a method for determining the effectiveness of cancer treatments with a RET tyrosine kinase inhibitor targeting said polynucleotides or polypeptides. This invention further relates to a method for cancer treatment using said effectiveness determination. Furthermore, this invention relates to agents for use in these methods.

### BACKGROUND ART

Gastric cancer is a cancer of which the second largest number of people in the world die, and is one of cancers from which many patients suffer in East Asia. The prognosis of this cancer has improved particularly in Japan due to the progress in early diagnostic technologies including endoscopy. However, patients with advanced or recurrent gastric cancer are still difficult to treat, and the 5-year survival rate of patients with this cancer in many countries including Europe and the United States is not more than 30%.

Gastric cancer is histopathologically classified into the following two major types: intestinal-type and diffuse-type (according to Lauren classification). The latter type of cancer, which is common in young people, easily develops peritoneal dissemination or lymph node metastasis, and has poor prognosis. The principal method adopted for treating gastric cancer is surgical operation, but patients treated with this method tend to easily develop peritoneal dissemination or metastases to other organs, so the diffuse-type gastric cancer associated with a high recurrence rate is difficult to treat with surgical operation. In addition, there has been developed no anticancer drug effective against gastric cancer, including diffuse-type cancer. Thus, to date, no therapeutic method has been established which is effective for gastric cancer, in particular diffuse-type gastric cancer.

Regarding gastric cancer, there have been reports on amplification of the EGFR, FGFR2, MET, ERBB2 and MYC genes, mutation of the KRAS, TP53 and CDH1 genes, and other alternations. At present, various molecular targeted therapies for gastric cancer have been studied which target the EGFR, FGFR2, ERBB2 or other gene. Thus, in the field of various cancers including gastric cancer, there is a strong demand for identifying oncogenes involved in the onsets of such cancers, such as mutant genes (mutant proteins) and fusion genes (fusion proteins), because such an identification will greatly contribute to development of novel cancer treatment and testing methods targeting such genes.

As regards other cancers, it has been reported that activating mutations of the RET gene occur in familial and sporadic thyroid cancer, some cases of colon cancer, and pheochromocytoma. It was also found that the RET gene is a responsible gene for multiple endocrine neoplasia (MEN) syndrome (MEN2A, MEN2B) (Non-patent Document 1). Further, the PTC-RET fusion gene was reported as a genomic aberration characteristic of thyroid cancer (Non-patent Document 2), and the presence of this type of gene was recently observed in lung cancer as well (Non-patent Document 3). Furthermore, the presence of the KIF5B-RET fusion gene has been found as a genomic aberration in lung adenocarcinoma (Non-patent Documents 3-5).

As low-molecular-weight inhibitors for the RET gene, vandatinib, motesanib, sorafenib, sunitinib, XL-184 and the like are known, and some of them are now being clinically developed as anticancer drugs.

In addition, various studies are made regarding using the foregoing mutant and fusion genes as indicators for predicting the effectiveness of treatments of cancers with said inhibitors. For example, it has been shown that tyrosine kinase inhibitors targeting EGFR or ALK protein are particularly effective for the treatment of lung adenocarcinoma harboring EGFR mutations and/or ALK fusions. Further, a technique for detecting a fusion of the ALK tyrosine kinase gene as observed in 4-5% of lung cancer cases was developed and clinically tested as a method to screen for cases indicated for inhibitors against ALK protein tyrosine kinase.

On the other hand, the CEP55 gene is found to be a microtubule-associated molecule which plays an important role in cytokinesis (Non-patent Document 6). This protein has multiple coiled coil domains including N-terminal region, and is presumed to be capable of dimerizing. Also, CEP55 protein is highly expressed in a wide variety of cancer cells, but is expressed at a low level in normal tissues, except that it is highly expressed in normal testis. Thus, CEP55 protein is known as a so-called cancer-testis antigen and is studied as a target for cancer vaccine therapies (Non-patent Document 7).

However, a thorough elucidation of fusion genes and other genes in various cancers including gastric cancer has not yet been achieved, and there is at present a demand for identifying fusion genes and other genes which contribute greatly to development of novel cancer treatment and testing methods and can also serve as indicators for predicting the effectiveness of drug treatments.

### CITATION LIST

### NON-PATENT DOCUMENTS

Non-patent Document 1: Kouvaraki MA., et al., Thyroid, 2005, vol. 15, p. 531-544
Non-patent Document 2: Nikiforov YE., et al., Nat Rev Endocrinol., 2011, vol. 7, p. 569-580
Non-patent Document 3: Takeuchi K., et al., Nat Med., 2012, vol. 18, p. 378-381
Non-patent Document 4: Kohno T., et al., Nat Med., 2012, vol. 18, 375-377
Non-patent Document 5: Lipson D., et al., Nat Med., 2012, vol. 18, p. 382-384
Non-patent Document 6: Zhao WM., et al., Mol Biol Cell., 2006, vol. 17, p. 3881-3896
Non-patent Document 7: Inoda S., et al., J Immunother., 2009, vol. 32, p. 474-485

### SUMMARY OF INVENTION

### TECHNICAL PROBLEM

The present invention has been made in consideration of the above-described problems with the prior art, and has as its object to identify genes that can serve as indicators for predicting the effectiveness of drug treatments in gastric cancer and other cancers. Another object of this invention is to provide novel methods for predicting the effectiveness of drug treatments targeting said genes and expression products thereof Still another object of this invention is to provide methods for treating gastric cancer and other cancers on the basis of the prediction of the effectiveness of drug treatments targeting said genes and expression products thereof. Yet another object of this invention is to provide agents for use in detecting said genes and expression products thereof in these methods.

### SOLUTION TO PROBLEM

As a result of intensive studies to achieve the above-mentioned objects, the present inventors have identified in-frame fusion transcripts between the CEP55 gene and the RET gene by performing transcriptome sequencing of 13 diffuse-type gastric cancer (DGC) specimens. Thus, it is considered that the fusion gene between the CEP55 gene and the RET gene (CEP55-RET fusion gene) is generated by reciprocal translocation between two human chromosomes 10 or by breakage and reunion in human chromosome 10. In fact, the inventors have investigated the nucleotide sequences of genomic fusion sites, and as a result, have found cases of fusion generated by breakage and reunion in human chromosome 10.

Thus, the inventors have introduced the CEP55-RET gene into normal cells, and have observed that the cells acquire anchorage-independent colony-forming ability, in other words become cancerous. We also have found that in these cells, the RET protein kinase is activated and also phosphorylation of its downstream signals such as AKT is increased.

Further, the inventors have subcutaneously transplanted CEP55-RET fusion gene-expressing cells into nude mice and, as a result, have observed that said cells have *in vivo* tumorigenic ability.

On the other hand, it has also been demonstrated that the activation of RET protein kinase, the phosphorylation of AKT and the like, the anchorage-independent colony-forming ability, and the *in vivo* tumorigenic ability are significantly suppressed by using a RET tyrosine kinase inhibitor or through inactivation of the kinase domain of the CEP55-RET fusion polypeptide.

On the basis of the above-described findings, the present inventors have found that it is possible to predict the effectiveness of treatment with a drug targeting this gene fusion in diffuse-type gastric cancer and other cancers, and that efficient treatments can be achieved by administering the drug to patients in whom the treatments with the drug have been determined to be effective on the basis of this prediction; thus, the inventors have completed the present invention.

Therefore, the present invention relates to polynucleotides encoding fusion polypeptides between CEP55 protein or part thereof and RET protein or part thereof, polypeptides encoded by said polynucleotides, a method for detecting said polynucleotides or polypeptides, a method for determining the effectiveness of cancer treatments with a RET tyrosine kinase inhibitor using the presence of said polynucleotides or polypeptides as an indicator, a method for treatment of cancer utilizing said effectiveness determination, and agents for use in these methods. More specifically, this invention provides the following:
[1] A polynucleotide encoding a polypeptide in which CEP55 protein or part thereof and RET protein or part thereof are fused together.
[2] A polypeptide encoded by the polynucleotide as set forth in [1].
[3] A method for detecting the presence or absence in a sample of the polynucleotide as set forth in [1] or of the polypeptide as set forth in [2], the method comprising the steps of:
   (a) contacting the sample with an agent intended for specifically detecting the presence or absence of the polynucleotide or the polypeptide in the sample; and
   (b) detecting the presence or absence of the polynucleotide or the polypeptide.
[4] An agent for detecting the presence or absence in a sample of the polynucleotide as set forth in [1] or of the polypeptide as set forth in [2] by the method as set forth in [3], the agent comprising a polynucleotide or polynucleotides as set forth below in any one of (a) to (c), the polynucleotide or polynucleotides having a chain length of at least 15 nucleotides, or an antibody as set forth below in (d):
   (a) a polynucleotide or polynucleotides that are at least one probe selected from the group consisting of a probe that hybridizes to a polynucleotide encoding CEP55 protein and a probe that hybridizes to a polynucleotide encoding RET protein;
   (b) a polynucleotide that is a probe that hybridizes to a point of fusion between a polynucleotide encoding CEP55 protein and a polynucleotide encoding RET protein;
   (c) polynucleotides that are a pair of primers designed to sandwich a point of fusion between a polynucleotide encoding CEP55 protein and a polynucleotide encoding RET protein; and
   (d) an antibody that binds to a polypeptide in which CEP55 protein and RET protein are fused together.
[5] A method for determining the effectiveness of a cancer treatment with a RET tyrosine kinase inhibitor, the method comprising the step of detecting the presence or absence in a sample isolated from a patient of the polynucleotide as set forth in [1] or of the polypeptide as set forth in [2], wherein in a case where the presence of the polynucleotide or the polypeptide is detected, the cancer treatment with the RET inhibitor is determined to be highly effective in the patient.
[6] An agent for determining the effectiveness of a cancer treatment with a RET inhibitor by the method as set forth in [5], the agent comprising a polynucleotide or polynucleotides as set forth below in any one of (a) to (c), the polynucleotide or polynucleotides having a chain length of at least 15 nucleotides, or an antibody as set forth below in (d):
   (a) a polynucleotide or polynucleotides that are at least one probe selected from the group consisting of a probe that hybridizes to a polynucleotide encoding CEP55 protein and a probe that hybridizes to a polynucleotide encoding RET protein;
   (b) a polynucleotide that is a probe that hybridizes to a point of fusion between a polynucleotide encoding CEP55 protein and a polynucleotide encoding RET protein;
   (c) polynucleotides that are a pair of primers designed to sandwich a point of fusion between a polynucleotide encoding CEP55 protein and a polynucleotide encoding RET protein; and
   (d) an antibody that binds to a polypeptide in which CEP55 protein and RET protein are fused together.
[7] A method for treatment of cancer, comprising the step of administering a RET tyrosine kinase inhibitor to a patient in whom a cancer treatment with the RET tyrosine kinase inhibitor has been determined to be highly effective by the method as set forth in [5].
[8] A therapeutic agent for cancer, comprising a RET tyrosine kinase inhibitor as an active ingredient, wherein the therapeutic agent is to be administered to a patient in whom a cancer treatment with the RET tyrosine kinase inhibitor has been determined to be highly effective by the method as set forth in [5].

### ADVANTAGEOUS EFFECTS OF INVENTION

The present invention enables effective detection of fusion genes between the CEP55 gene and the RET gene, and expression products thereof This invention also makes it possible to predict the effectiveness of various treatments on cancers, in particular the effectiveness of cancer treatments with a RET tyrosine kinase inhibitor, on the basis of the detection of said fusion genes and expression products thereof This prediction makes it possible to avoid administration of a drug to cancer patients conceivably not responsive to the administration of the drug, thereby allowing efficient cancer treatments.

### BRIEF DESCRIPTION OF DRAWINGS

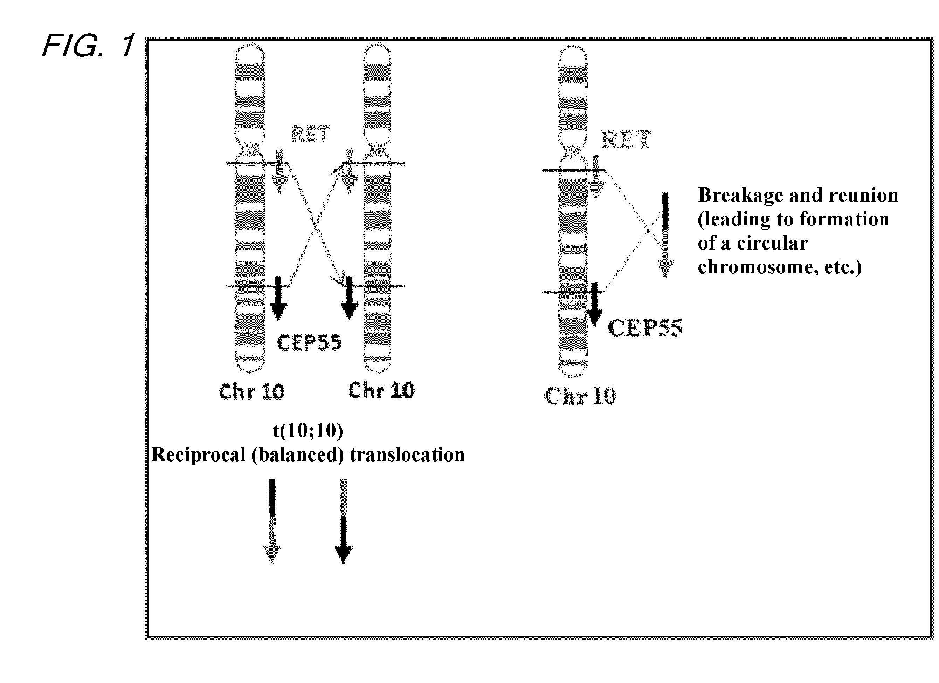

FIG. 1 is a schematic diagram showing modes of the fusion between the CEP55 gene and the RET gene. The left half of this figure shows one mode of the CEP55-RET fusion, which is generated by reciprocal translocation between two human chromosomes 10, and the right half of this figure shows another mode of fusion which is generated by breakage and reunion in human chromosome 10.

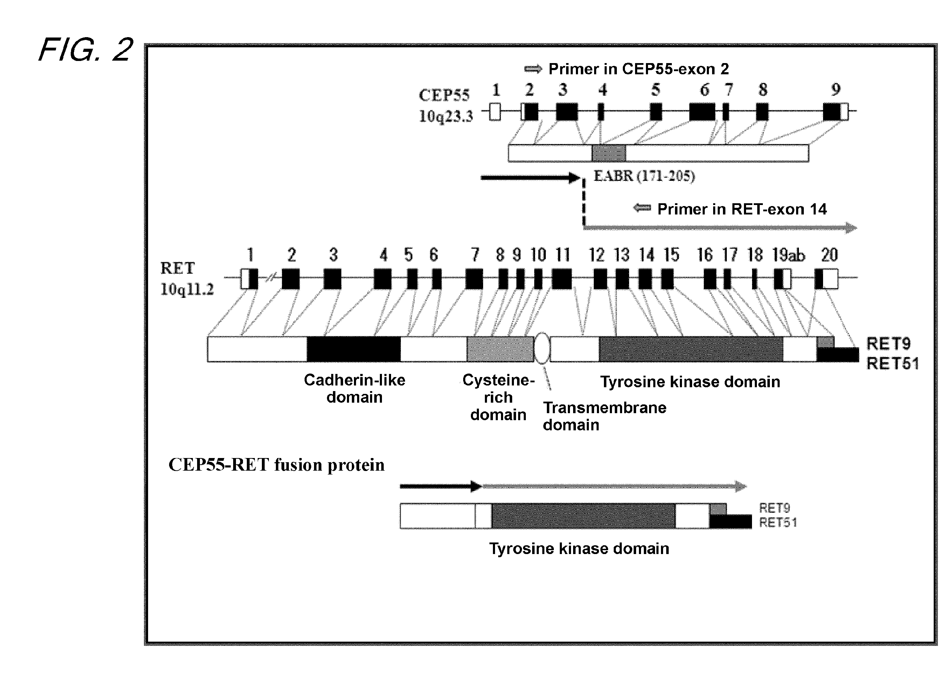

FIG. 2 is a schematic diagram showing the fusion between CEP55 protein and RET protein. More specifically, this schematic diagram shows that the N-terminal moiety of CEP55 protein is fused with the C-terminal moiety of RET protein, which comprises a tyrosine kinase domain. This diagram also shows that a polynucleotide encoding a fusion polypeptide between CEP55 protein and RET protein can be detected by using RT-PCR primers each hybridizing to exon 2 in the CEP55 gene or exon 14 in the RET gene.

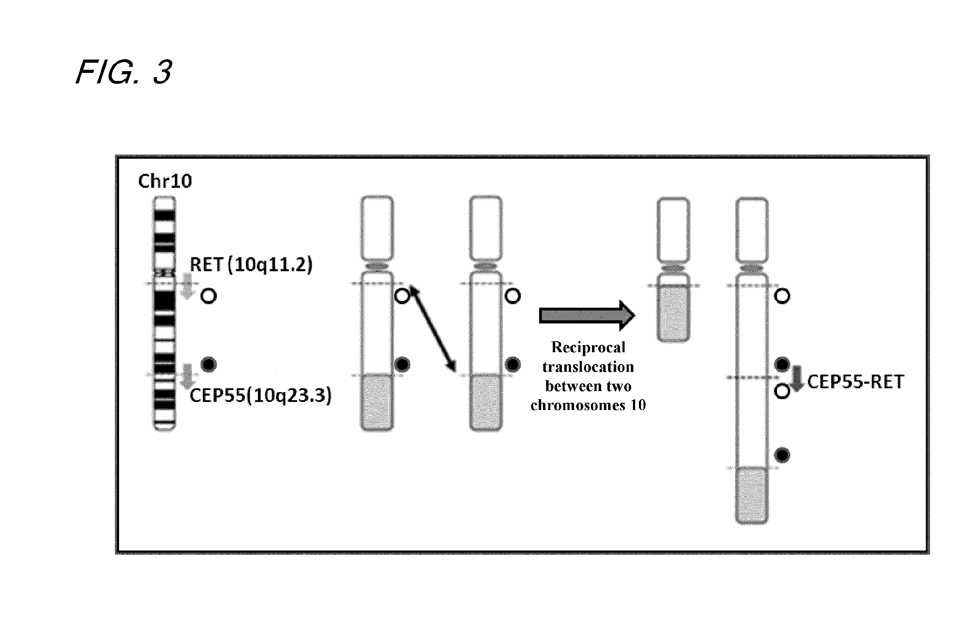

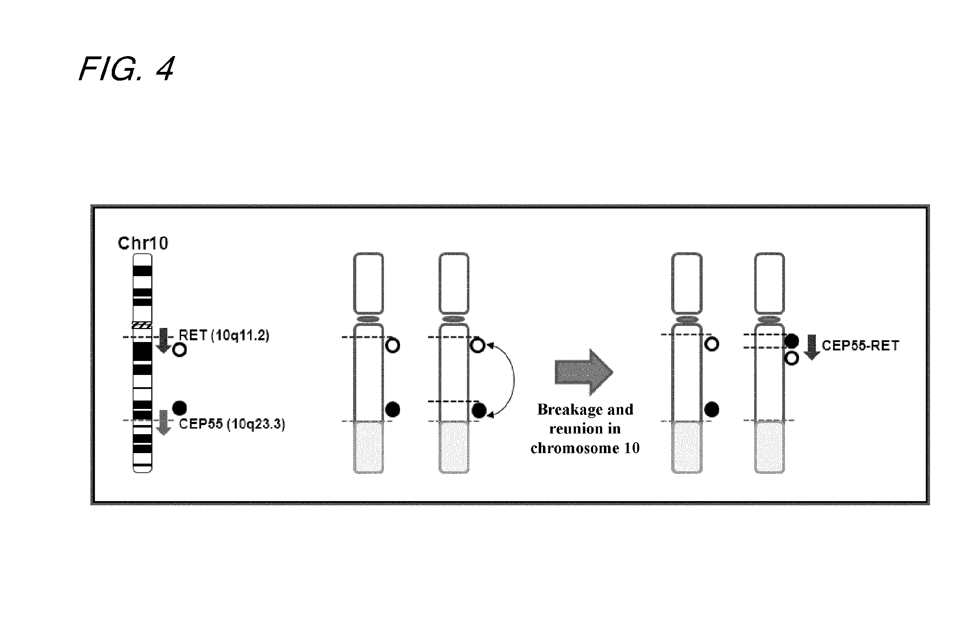

FIG. 3 is a schematic diagram showing a method for detecting the inventive fusion gene by the ISH method. More specifically, this schematic diagram shows that in the case of the CEP55-RET fusion gene, the presence of the CEP55-RET fusion gene generated by reciprocal translocation between two human chromosomes 10 can be detected by designing ISH probes each specific for a portion toward the 5' end of the CEP55 gene or for a portion toward the 3' end of the RET gene. In this figure, the black circle represents a probe hybridizing to a portion toward the 5' end of the CEP55 gene, and the white circle represents a probe hybridizing to a portion toward the 3' end of the RET gene (also in FIG. 4, probes are represented in the same way).
FIG. 4 is a schematic diagram showing a method for detecting the inventive fusion gene by the ISH method. More specifically, this schematic diagram shows that in the case of the CEP55-RET fusion gene, the presence of the CEP55-RET fusion gene generated by breakage and reunion in human chromosome 10 can be detected by designing ISH probes each specific for a portion toward the 5' end of the CEP55 gene or for a portion toward the 3' end of the RET gene.

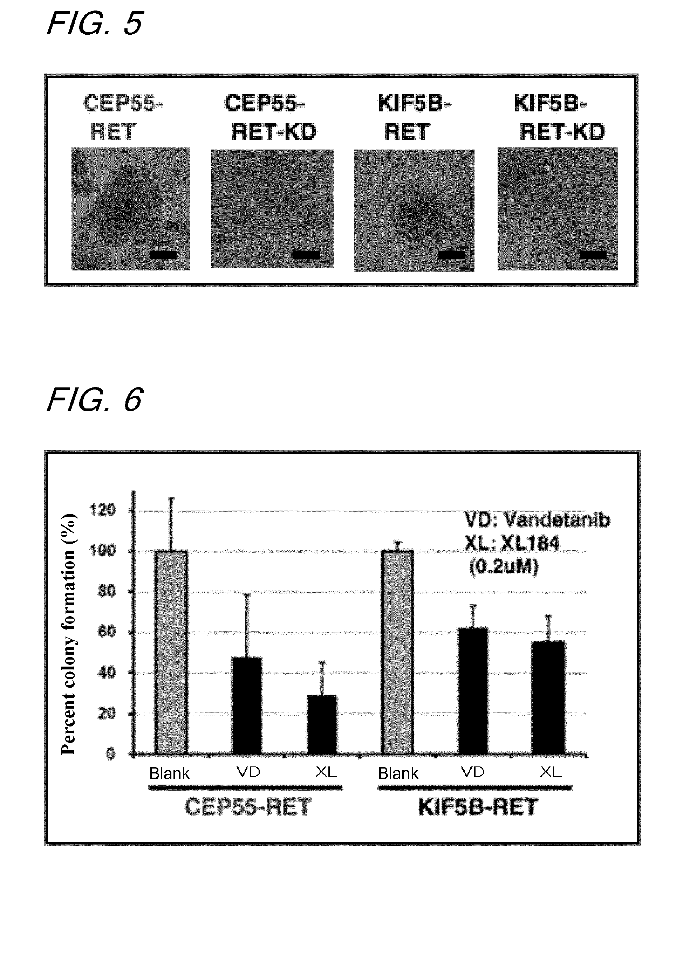

FIG. 5 is a set of photos showing the results of observing, under a microscope, the anchorage-independent colony formation in normal murine fibroblast lines each stably expressing the wild-type or the mutated RET fusion polypeptide, which were each seeded in a soft agar medium. In this figure, the "CEP55-RET" panel shows the result for a cell line expressing the wild-type CEP55-RET fusion polypeptide, the "CEP55-RET-KD" panel shows the result for a cell line expressing the mutated CEP55-RET fusion polypeptide, the "KIF5B-RET" panel shows the result for a cell line expressing the wild-type KIF5B-RET fusion polypeptide, and the "KIF5B-RET-KD" panel shows the result for a cell line expressing the mutated KIF5B-RET fusion polypeptide. The bars in these panels indicate the length of 100 µm.
FIG. 6 is a graph showing the results of analyzing the anchorage-independent colony-forming ability of normal murine fibroblast lines stably expressing any one of the wild-type RET fusion polypeptides, which were each seeded in a soft agar supplemented with a RET tyrosine kinase inhibitor (vandetanib or XL184). In this graph, "CEP55-RET" represents the results for cell lines expressing the wild-type CEP55-RET fusion polypeptide, and "KIF5B-RET" represents the results for cell lines expressing the wild-type KIF5B-RET fusion polypeptide. The "VD" bars show the results of colony formation in the presence of vandetanib (at a vandetanib concentration in medium of 0.2 µM), the "XL" bars show the results of colony formation in the presence of XL184 (at an XL184 concentration in medium of 0.2 µM), and the "Blank" bars show the results of colony formation in the absence of a RET tyrosine kinase inhibitor. The vertical axis represents relative values calculated with respect to the number of colonies formed in each of the cell lines expressing any one of the wild-type RET fusion polypeptides in the absence of a RET tyrosine kinase inhibitor, which is taken as 100.

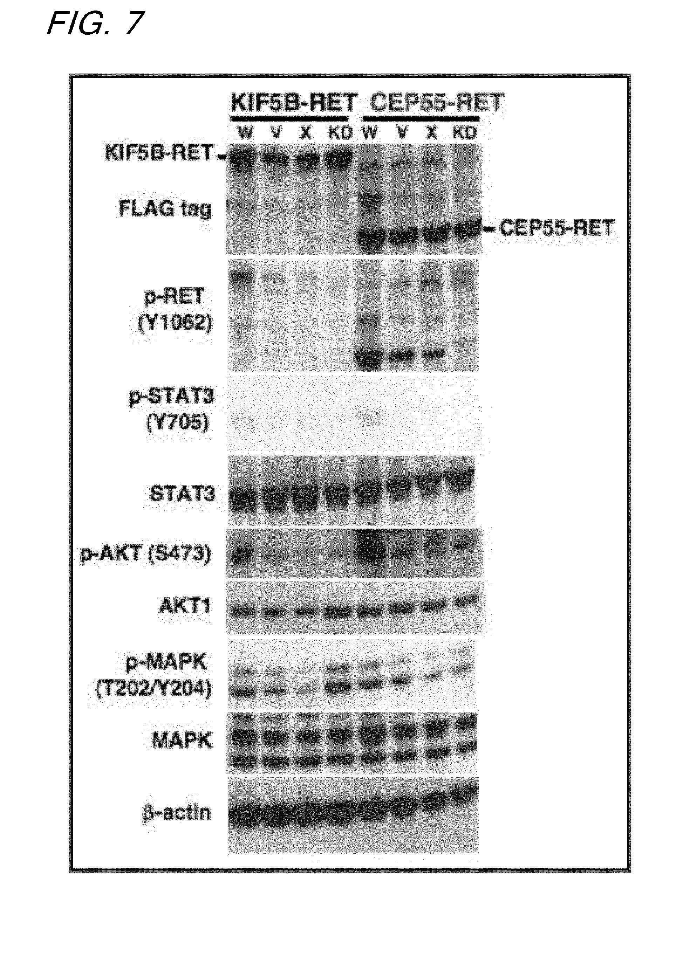

FIG. 7 is a set of photos showing the results of analyzing by Western blotting the phosphorylation of various proteins in normal murine fibroblast lines stably expressing any one of the RET fusion polypeptides, which were subjected to serum starvation and then cultured in the presence of a RET tyrosine kinase inhibitor (vandetanib or XL184). In this figure, "CEP55-RET" represents the results for cell lines expressing the CEP55-RET fusion polypeptide, and "KIF5B-RET" represents the results for cell lines expressing the KIF5B-RET fusion polypeptide. The lanes labeled as "W" show the results of culturing the wild-type RET fusion polypeptide-expressing cell lines in the absence of a RET tyrosine kinase inhibitor; the lanes labeled as "V" show the results of culturing the wild-type RET fusion polypeptide-expressing cell lines in the presence of vandetanib (at a vandetanib concentration in medium of 1 µM); the lanes labeled as "X" show the results of culturing the wild-type RET fusion polypeptide-expressing cell lines in the presence of XL184 (at a XL184 concentration in medium of 1 µM); and the lanes labeled as "KD" show the results of culturing the mutated RET fusion polypeptide-expressing cell lines in the absence of a RET tyrosine kinase inhibitor. Since these fusion polypeptides were further tagged with the FLAG tag and expressed intracellularly, the "FLAG tag" row in this figure shows the results of detecting the respective fusion polypeptides through this tag. The "p-RET (Y1062)" row shows the results of detecting phosphorylated RET. The "STAT3" and "p-STAT3 (Y705)" rows show the results of detecting STAT3 and phosphorylated STAT3, respectively. The "AKT" and "p-AKT (S473)" show the results of detecting AKT and phosphorylated AKT, respectively. The "MAPK" and "p-MAPK (T202/Y204)" rows show the results of detecting MAPK and phosphorylated MAPK, respectively. The "β-actin" row shows that the amount of protein used as an internal standard is uniform among the lanes.

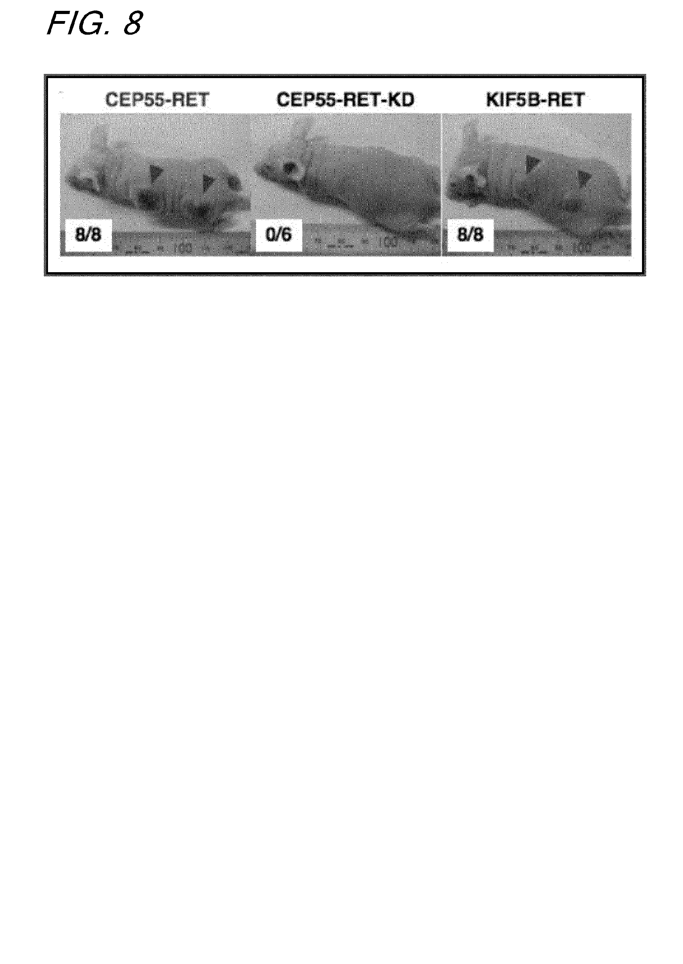

FIG. 8 is a set of photos showing the results of subcutaneously transplanting each of normal murine fibroblast lines expressing any of the RET fusion polypeptides into immunodeficient mice. In this figure, the "CEP55-RET" panel is a photo taken upon the observation of an immunodeficient mouse 18 days after it was transplanted subcutaneously with the cell line expressing the wild-type CEP55-RET fusion polypeptide. The "KIF5B-RET" panel is a photo taken upon the observation of an immunodeficient mouse 18 days after it was transplanted subcutaneously with the cell line expressing the wild-type KIF5B-RET fusion polypeptide. Triangles indicate formed tumors. "8/8" represents that the cells of interest were transplanted into two sites of each of four mice, a total of eight sites, and as a result, tumorigenesis was observed in all of the eight sites. The "CEP55-RET-KD" panel is a photo taken upon the observation of an immunodeficient mouse 18 days after it was transplanted subcutaneously with the cell line expressing the mutated CEP55-RET fusion polypeptide. "0/6" represents that the cells of interest were transplanted into two sites of each of three mice, a total of six sites, and as a result, no tumorigenesis was observed in any of the six sites.

### DESCRIPTION OF EMBODIMENTS

### <Polynucleotides encoding fusion polypeptides between CEP55 protein and RET protein, and polypeptides encoded by the polynucleotides>

As disclosed below in Examples, multiple cases of fusion between the CEP55 gene and the RET gene were first discovered according to the present invention. Therefore, this invention provides a polynucleotide encoding a fusion polypeptide between CEP55 protein or part thereof and RET protein or part thereof (said polynucleotide and said fusion polypeptide are to be hereinafter also referred to as the "CEP55-RET fusion polynucleotide" and the "CEP55-RET fusion polypeptide", respectively).

The "CEP55-RET fusion polypeptide" as referred to in the present invention means a polypeptide in which the full length or part of CEP55 protein is fused with the full length or part of RET protein. Also, the "CEP55-RET fusion polynucleotide" as referred to in this invention means a polynucleotide in which a polynucleotide encoding the full length or part of CEP55 protein is fused with a polynucleotide encoding the full length or part of RET protein.

The "CEP55 (centrosomal protein 55 kDa) protein" according to the present invention refers to a protein encoded by the gene located at a long arm of chromosome 10 (10q23.3) in humans. In this invention, the "CEP55 protein", as far as it is derived from humans, is typified by the protein consisting of the amino acid sequence of SEQ ID NO: 2. The polynucleotide encoding the CEP55 protein is typified by the polynucleotide consisting of the nucleotide sequence of SEQ ID NO: 1.

The "RET (rearranged during transfection) protein" according to the present invention is also referred to as "RET tyrosine kinase protein" or "RET receptor tyrosine kinase protein", and means a protein encoded by the gene located at 10q11.2 in humans. In this invention, the "RET protein", as far as it is derived from humans, is typified by the protein consisting of the amino acid sequence of SEQ ID NO: 4 (RET51, RET isoform a) and the protein consisting of the amino acid sequence of SEQ ID NO: 6 (RET9, RET isoform c). The polynucleotide encoding the RET protein is typified by the polynucleotide consisting of the nucleotide sequence of SEQ ID NO: 3 (RET transcript variant 2) and the polynucleotide consisting of the nucleotide sequence of SEQ ID NO: 5 (RET transcript variant 4). As shown in FIG. 2, the protein consisting of the amino acid sequence of SEQ ID NO: 4 (RET51) and the protein consisting of the amino acid sequence of SEQ ID NO: 6 (RET9) share the common amino acid sequence starting with methionine at position 1 and ending with glycine at position 1063, but differ from each other in the sequence and length of the C-terminal moiety.

The CEP55-RET fusion polynucleotide is typified by a polynucleotide encoding a polypeptide in which the N-terminal moiety of CEP55 protein is fused with the C-terminal moiety of RET protein.

In the present invention, the "N-terminal moiety of CEP55 protein" is typified by a moiety comprising a region starting with methionine at position 1 and ending with glutamine at position 153 in the CEP55 protein. And the "C-terminal moiety of RET protein" is typified by a moiety comprising a tyrosine kinase domain in RET protein (refer to FIG. 2).

In the present invention, the "polynucleotide encoding a polypeptide in which the N-terminal moiety of CEP55 protein is fused with the C-terminal moiety of RET protein" is typically generated, as shown in FIGs. 1 to 4, by reciprocal translocation between two human chromosomes 10 or by breakage and reunion in human chromosome 10. More specifically, this term refers to a polynucleotide encoding a polypeptide in which a polypeptide region of the CEP55 protein that is encoded by exons 1-3 is fused with a polypeptide region of the RET protein that is encoded by exons 12-19b or 12-20 (e.g., the polypeptide consisting of the amino acid sequence of SEQ ID NO: 8 or 10). Said polynucleotide is exemplified by the polynucleotide consisting of the nucleotide sequence of SEQ ID NO: 7 or 9.

In the present invention, the amino acid sequences of "CEP55 protein" and "RET protein", and the nucleotide sequences of the genes encoding said proteins can mutate in nature (i.e., in a non-artificial way). Thus, the amino acid sequence of the "CEP55-RET fusion polypeptide" and the nucleotide sequence of the "CEP55-RET fusion polynucleotide" can also mutate in nature (i.e., in a non-artificial way). Said amino acid sequences and nucleotide sequences may be artificially modified. Such mutants are also encompassed by this invention.

Certain exemplary mutants of the CEP55-RET fusion polypeptide include proteins consisting of an amino acid sequence derived from the amino acid sequence of SEQ ID NO: 8 or 10 by substitution, deletion, addition and/or insertion of one or more amino acids.

As used herein, the term "more" refers to generally 50 or fewer amino acids, preferably 30 or fewer amino acids, more preferably 10 or fewer amino acids, and particularly preferably several or fewer amino acids (for example, five or fewer amino acids, three or fewer amino acids, two or one amino acid, one amino acid).

Other exemplary mutants of the CEP55-RET fusion polypeptide include polypeptides encoded by a DNA that hybridizes under stringent conditions to a DNA consisting of the nucleotide sequence of SEQ ID NO: 7 or 9.

Exemplary high stringent hybridization conditions are 0.2×SSC at 65°C, and exemplary low stringent hybridization conditions are 2.0×SSC at 50°C.

Still other exemplary mutants of the CEP55-RET fusion polypeptide include polypeptides consisting of an amino acid sequence having at least 80% (for example, at least 85%, 90%, 95%, 97%, 99%) homology to the amino acid sequence of SEQ ID NO: 8.

Sequence homology can be determined using the BLASTX or BLASTP (amino acid level) program (Altschul, et al., J. Mol. Biol., 1990, 215: 403-410). This program is based on the algorithm BLAST developed by Karlin and Altschul (Proc. Natl. Acad. Sci. USA, 1990, 87: 2264-2268; and Proc. Natl. Acad. Sci. USA, 1993, 90: 5873-5877). When amino acid sequence analysis is performed using BLASTX, the parameters are typically set as follows: score = 50 and wordlength = 3. Amino acid sequence analysis using the Gapped BLAST program can be performed as per the descriptions in Altschul, et al. (Nucleic Acids Res., 1997, 25: 3389-3402). When amino acid sequence analysis is performed using the BLAST and Gapped BLAST programs, the default parameters of these programs are used. The specific procedures for conducting these analyses are known.

Exemplary mutants of the CEP55-RET fusion polynucleotide include polynucleotides encoding the above-mentioned mutants of the CEP55-RET fusion polypeptide, and polynucleotides encoding degenerate variants of said polypeptide which have no amino acid mutation.

Exemplary forms of the "CEP55-RET fusion polynucleotide" according to the present invention include mRNA, cDNA, and genomic DNA. It is possible for those skilled in the art using a known hybridization technique to isolate the "CEP55-RET fusion polynucleotide" from a cDNA library or genomic DNA library prepared from diffuse-type gastric cancer or other cancers that harbor a fusion gene between the CEP55 gene and the RET gene. The polynucleotide can also be prepared by amplification utilizing a known gene amplification technique (e.g., PCR), with the mRNA, cDNA or genomic DNA prepared from diffuse-type gastric cancer or other cancers being used as a template.

Furthermore, after the thus-prepared polynucleotide is inserted into an appropriate expression vector, the vector is introduced into a cell-free protein synthesis system (e.g., reticulocyte extract, wheat germ extract) and the system is incubated, or alternatively the vector is introduced into appropriate cells (e.g., *E coli.,* yeast, insect cells, animal cells) and the resulting transformant is cultured; in either way, the CEP55-RET fusion polypeptide can be prepared.

As mentioned above, the "CEP55-RET fusion polypeptide" and "CEP55-RET fusion polynucleotide" according to the present invention encompasses, in a broad sense, both those having naturally occurring sequences (including those mutated in nature) and those having artificially modified sequences. However, it should be noted that the "CEP55-RET fusion polypeptide" and "CEP55-RET fusion polynucleotide", particularly if these terms are used as an object of the detection as described below, mainly refer to those having naturally occurring sequences (including those mutated in nature).

### <Method for detecting the presence or absence of the CEP55-RET fusion polypeptide or the CEP55-RET fusion polynucleotide>

The present invention also provides a method for detecting the presence or absence of the CEP55-RET fusion polynucleotide or the CEP55-RET fusion polypeptide in a sample. The detection method of this invention comprises the steps of: (a) contacting the sample with an agent intended for specifically detecting the presence or absence of the polynucleotide or the polypeptide in the sample; and (b) detecting the presence or absence of the polynucleotide or the polypeptide.

For the purpose of the present invention, the term "sample" includes not only biological samples (for example, cells, tissues, organs, body fluids (e.g., blood, lymphs), digestive juices, sputum, bronchoalveolar/bronchial lavage fluids, urine, and feces), but also nucleic acid extracts from these biological samples (for example, genomic DNA extracts, mRNA extracts, and cDNA and cRNA preparations from mRNA extracts) and protein extracts. The sample may also be the one that is fixed with formalin or alcohol, frozen, or embedded in paraffin.

Further, the genomic DNA, mRNA, cDNA or protein can be prepared by those skilled in the art through considering various factors including the type and state of the sample and selecting a known technique suitable therefor.

In the present invention, "detecting the presence or absence of the CEP55-RET fusion polynucleotide or the CEP55-RET fusion polypeptide" can be performed on genomic DNAs encoding said fusion polypeptide, transcripts from said genomic DNAs, or translation products from said transcripts.

Since a genomic DNA encoding the CEP55-RET fusion polypeptide is formed by reciprocal translocation between two human chromosomes 10 or by breakage and reunion in human chromosome 10, "detecting the presence or absence of the CEP55-RET fusion polynucleotide" may be achieved by detecting this phenomenon of reciprocal translocation (refer to FIGs. 1 and 3). The detection of reciprocal translocation may be achieved, for example, by detecting a split of the portion consisting of the exon 3-coding region of the CEP55 gene and a region upstream from said coding region toward the 5' end, from the portion consisting of the exon 4-coding region of the CEP55 gene and a region downstream from said coding region toward the 3' end, or by detecting a split of the portion consisting of the exon 11-coding region of the RET gene and a region upstream from said coding region toward the 5' end, from the portion consisting of the exon 12-coding region of the RET gene and a region downstream from said coding region toward the 3' end.

"Detecting the presence of absence of the CEP55-RET fusion polynucleotide" according to the present invention can be performed using a known method. Exemplary known methods that can be used in the detection on the "genomic DNAs encoding said fusion polypeptide" include *in situ* hybridization (ISH) using fluorescence or other means, genomic PCR, direct sequencing, Southern blotting, and genome microarray analysis. Exemplary known methods that can be used in the detection on the "transcripts from said genomic DNAs" include RT-PCR, direct sequencing, Northern blotting, dot blotting, and cDNA microarray analysis.

According to *in situ* hybridization, genomic DNAs encoding the CEP55-RET fusion polypeptide can be detected by contacting a biological sample with the polynucleotide or polynucleotides noted below in (a) or (b), which have a chain length of at least 15 nucleotides:
(a) a polynucleotide or polynucleotides that are at least one probe selected from the group consisting of a probe that hybridizes to a polynucleotide encoding CEP55 protein and a probe that hybridizes to a polynucleotide encoding RET protein; or
(b) a polynucleotide that is a probe that hybridizes to a point of fusion between a polynucleotide encoding CEP55 protein and a polynucleotide encoding RET protein.

In relation to the detection of genomic DNAs encoding the CEP55-RET fusion polypeptide, the "polynucleotide encoding CEP55 protein" according to the present invention, as far as it is derived from humans, is typified by the gene consisting of the DNA sequence of positions 95256369 to 95288849 in the genome sequence identified by RefSeq ID: NC_000010.10 ("CEP55 gene").

The "polynucleotide encoding RET protein" according to the present invention, as far as it is derived from humans, is typified by the gene consisting of the DNA sequence of positions 43572517 to 43625799 in the genome sequence identified by RefSeq ID: NC_000010.10 ("RET gene").

However, the DNA sequences of the genes can vary in nature (i.e., in a non-artificial way) due to their mutations and the like. Thus, such naturally occurring mutants can also be encompassed by the present invention (the same applies hereinafter).

The polynucleotide(s) of (a) according to the present invention can be of any type as far as it is capable of detecting the presence of a genomic DNA encoding the CEP55-RET fusion polypeptide in the foregoing biological sample by hybridizing to a nucleotide sequence targeted by said polynucleotide, or more specifically to a polynucleotide encoding CEP55 protein or a polynucleotide encoding RET protein. The polynucleotide(s) of (a) is preferably any of the polynucleotides noted below in (a1) to (a3):
(a1) a combination of a polynucleotide that hybridizes to the portion consisting of the exon 3-coding region of the CEP55 gene and a region upstream from said coding region toward the 5' end (this polynucleotide is to be hereinafter also referred to as "5' CEP55 probe"), and a polynucleotide that hybridizes to the portion consisting of the exon 12-coding region of the RET gene and a region downstream from said coding region toward the 3' end (this polynucleotide is to be hereinafter also referred to as "3' RET probe");
(a2) a combination of 5' CEP55 probe and a polynucleotide that hybridizes to the portion consisting of the exon 4-coding region of the CEP55 gene and a region downstream from said coding region toward the 3' end (this polynucleotide is to be hereinafter also referred to as "3' CEP55 probe"); and
(a3) a combination of a polynucleotide that hybridizes to the portion consisting of the exon 11-coding region of the RET gene and a region upstream from said coding region toward the 5' end (this polynucleotide is to be hereinafter also referred to as "5' RET probe"), and 3' RET probe.

In the present invention, the region to which the pair of polynucleotides of (a1) as used *for in situ* hybridization is to hybridize (such a region is to be hereinafter referred to as the "target nucleotide sequence") is preferred to be a region extending for not more than 1000000 nucleotides from a point of fusion between a polynucleotide encoding CEP55 protein and a polynucleotide encoding RET protein, from the viewpoints of specificity for the target nucleotide sequence and detection sensitivity. And the region to which the pair of polynucleotides of (a2) or (a3) as used *for in situ* hybridization is to hybridize is preferred, from the same viewpoints, to be a region extending for not more than 1000000 nucleotides from a breakpoint in a polynucleotide encoding CEP55 protein or in a polynucleotide encoding RET protein.

In the present invention, the polynucleotide of (b) as used *for in situ* hybridization can be of any type as far as it is capable of detecting the presence of a genomic DNA encoding the CEP55-RET fusion polypeptide in the foregoing biological sample by hybridizing to a nucleotide sequence targeted by said polynucleotide, or more specifically to a point of fusion between a polynucleotide encoding CEP55 protein and a polynucleotide encoding RET protein. Typical examples of the polynucleotide of (b) are those which hybridize to a genomic DNA encoding a polynucleotide consisting of the nucleotide sequence of SEQ ID NO: 7 or 9, for example, those which hybridize to a point of fusion between a polynucleotide encoding CEP55 protein and a polynucleotide encoding RET protein.

Further, in the present invention, the polynucleotide or polynucleotides of (a) or (b) as used *for in situ* hybridization are preferred to be a group consisting of multiple types of polynucleotides which can cover the entire target nucleotide sequence, from the viewpoints of further improvement of specificity for the target nucleotide sequence and detection sensitivity. In such a case, the polynucleotides constituting said group each have a length of at least 15 nucleotides, preferably from 100 to 1000 nucleotides.

The polynucleotide or polynucleotides of (a) or (b) as used for *in situ* hybridization are preferably labeled with a fluorescent dye or other means for the purpose of detection. Examples of such a fluorescent dye include, but are not limited to, DEAC, FITC, R6G, TexRed, and Cy5. Aside from fluorescent dyes, the polynucleotide may also be labeled with a dye (chromogen) such as DAB or with silver or other means based on enzymatic metal deposition.

In the process of *in situ* hybridization, a probe specific for a polynucleotide encoding CEP55 protein and a probe specific for a polynucleotide encoding RET protein are preferably each labeled with a different dye. If *in situ* hybridization is carried out using the probe combination of (a1) which consists of probes labeled with different dyes, to thereby observe an overlap between signals emitted from the labels on these probes, then it can be determined that a genomic DNA encoding the CEP55-RET fusion polypeptide has been detected successfully (refer to FIGs. 3 and 4). Also, if *in situ* hybridization is carried out using the probe combination of (a2) or (a3) which consists of probes labeled with different dyes, to thereby observe a split between signals emitted from the labels on these probes, then it can be determined that a genomic DNA encoding the CEP55-RET fusion polypeptide has been detected successfully.

Polynucleotide labeling can be effected by a known method. For example, the polynucleotides can be labeled by nick translation or random priming, in which the polynucleotides are caused to incorporate substrate nucleotides labeled with a fluorescent dye or other means.

The conditions for contacting the foregoing biological sample with the polynucleotide(s) of (a) or (b) in the process of *in situ* hybridization can vary with various factors including the length of said polynucleotide(s); and exemplary high stringent hybridization conditions are 0.2×SSC at 65°C, and exemplary low stringent hybridization conditions are 2.0×SSC at 50°C. Those skilled in the art could realize comparable stringent hybridization conditions to those mentioned above, by appropriately selecting salt concentration (e.g., SSC dilution rate), temperature, and various other conditions including concentrations of surfactant (e.g., NP-40) and formamide, and pH.

Aside from *in situ* hybridization, other examples of the method for detecting a genomic DNA encoding the CEP55-RET fusion polypeptide using the polynucleotide(s) of (a) or (b) include Southern blotting, Northern blotting and dot blotting. According to these methods, the fusion gene is detected by hybridizing the polynucleotide(s) of (a) or (b) to a membrane in which a nucleic acid extract from the foregoing biological sample has been transcribed. In the case of using the polynucleotide(s) of (a), if a polynucleotide that hybridizes to a polynucleotide encoding CEP55 protein and a polynucleotide that hybridizes to a polynucleotide encoding RET protein recognize the same band present in the membrane, then it can be determined that a genomic DNA encoding the CEP55-RET fusion polypeptide has been detected successfully.

Additional examples of the method for detecting a genomic DNA encoding the CEP55-RET fusion polypeptide using the polynucleotide of (b) include genome microarray analysis and DNA microarray analysis. According to these methods, the genomic DNA is detected by preparing an array in which the polynucleotide of (b) is immobilized on a substrate and bringing the foregoing biological sample into contact with the polynucleotide immobilized on the array.

In the process of PCR or sequencing, the polynucleotides noted below in (c) can be used to specifically amplify part or all of the CEP55-RET fusion polynucleotide using a DNA (genomic DNA, cDNA) or RNA prepared from the foregoing biological sample as a template:
(c) polynucleotides that are a pair of primers designed to sandwich a point of fusion between a polynucleotide encoding CEP55 protein and a polynucleotide encoding RET protein.

The "polynucleotides that are a pair of primers" refers to a primer set designed such that in the foregoing fusion polynucleotide or the like to be targeted, one of the primers hybridizes to a region of the CEP55 gene and the other primer hybridizes to a region of the RET gene. These polynucleotides have a length of generally 15-100 nucleotides, preferably 17-30 nucleotides.

Also, it is preferred from the viewpoints of the accuracy and sensitivity of PCR detection that the polynucleotides of (c) according to the present invention should each consist of a sequence complementary to the nucleotide sequence of said fusion polynucleotide which extends for not more than 5000 nucleotides from a point of fusion between a polynucleotide encoding CEP55 protein and a polynucleotide encoding RET protein.

The "polynucleotides that are a pair of primers" can be designed by a known method as appropriate based on the nucleotide sequence of the CEP55-RET fusion polynucleotide or the like to be targeted. Exemplary known methods include a method using the Primer Express® software (ABI).

Preferred examples of the "polynucleotides that are a pair of primers" are preferably the polynucleotides noted below in (c1):
(c1) a combination of a polynucleotide that hybridizes to the portion consisting of the exon 3-coding region of the CEP55 gene and a region upstream from said coding region toward the 5' end (this polynucleotide is to be hereinafter also referred to as "5' CEP55 primer"), and a polynucleotide that hybridizes to the portion consisting of the exon 12-coding region of the RET gene and a region downstream from said coding region toward the 3' end (this polynucleotide is to be hereinafter also referred to as "3' RET primer").

For example, in the process of detection of the CEP55-RET fusion polynucleotide, primers are each designed for exon 2 containing the start codon of the CEP55 gene or for exon 14 in the kinase region of the RET gene, so that detection can be made of all variants containing the tyrosine kinase region of RET protein, and all fusion genes with the CEP55 gene (refer to FIG. 2).

In the present invention, the method for detecting a translation product of the CEP55-RET fusion polynucleotide can be exemplified by immunostaining, Western blotting, ELISA, flow cytometry, immunoprecipitation, and antibody array analysis. These methods use an antibody binding to the CEP55-RET fusion polypeptide. Examples of such an antibody include an antibody specific to a polypeptide containing a point of fusion between CEP55 protein and RET protein (hereinafter also referred to as the "fusion point-specific antibody"), an antibody binding to a polypeptide consisting of a region of CEP55 protein that extends toward the N-terminus with respect to said point of fusion (hereinafter also referred to as the "CEP55-N terminal antibody"), and an antibody binding to a polypeptide consisting of a region of RET protein that extends toward the C-terminus with respect to said point of fusion (hereinafter also referred to as the "RET protein-C terminal antibody"). As referred to herein, the "fusion point-specific antibody" means an antibody that specifically binds to a polypeptide containing said point of fusion but does not bind to either wild-type (normal) CEP55 protein or wild-type (normal) RET protein.

The CEP55-RET fusion polypeptide can be detected by the fusion point-specific antibody or a combination of the CEP55-N terminal antibody and the RET protein-C terminal antibody.

The "antibody binding to the CEP55-RET fusion polypeptide" can be prepared by those skilled in the art through selection of a known method as appropriate. Examples of such a known method include: a method in which the polypeptide comprising the C-terminal moiety of RET protein, the CEP55-RET fusion polypeptide, the polypeptide comprising the N-terminal moiety of CEP55 protein, and/or the like are inoculated into an immune animal, the immune system of the animal is activated, and then the serum (polyclonal antibody) of the animal is collected; as well as monoclonal antibody preparation methods such as hybridoma method, recombinant DNA method, and phage display method. If an antibody having a labeling agent attached thereto is used, a target protein can be directly detected by detecting this label. The labeling agent is not particularly limited as long as it is capable of binding to an antibody and is detectable, and examples include peroxidase, β-D-galactosidase, microperoxidase, horseradish peroxidase (HRP), fluorescein isothiocyanate (FITC), rhodamine isothiocyanate (RITC), alkaline phosphatase, biotin, and radioactive materials. Aside from the direct detection of a target protein using an antibody having a labeling agent attached thereto, the target protein can also be indirectly detected using a secondary antibody having a labeling agent attached thereto, Protein G or A, or the like.

### <Method for determining the effectiveness of cancer treatments with a RET tyrosine kinase inhibitor>

As disclosed below in Examples, gene fusions between the CEP55 gene and the RET gene are believed to induce activation of RET protein and contribute to malignant transformation of cancers and other pathological conditions. Thus, it is highly probable that cancer patients with detection of such a fusion are responsive to treatments with a RET tyrosine kinase inhibitor.

Therefore, the present invention provides a method for determining the effectiveness of a cancer treatment with a RET tyrosine kinase inhibitor, the method comprising the step of detecting the presence or absence of the CEP55-RET fusion polynucleotide or the CEP55-RET fusion polypeptide in a sample isolated from a patient, wherein in a case where the presence of the polynucleotide or polypeptide is detected, the cancer treatment with the RET tyrosine kinase inhibitor is determined to be highly effective in the patient.

For the purpose of the present invention, the "patient" can be not only a human suffering from a cancer but also a human suspected of having a cancer. The "cancer" to which the method of this invention is to be applied is not particularly limited as long as it is a cancer with expression of the CEP55-RET fusion gene. The cancer is preferably diffuse-type gastric cancer. Collection of a biological sample from a patient can be performed by a known method depending on the type of the biological sample.

For the purpose of the present invention, the "RET tyrosine kinase inhibitor", the cancer treatment with which is to be evaluated for effectiveness, is not particularly limited as long as it is a substance capable of directly or indirectly suppressing the function of RET protein. Examples of known RET tyrosine kinase inhibitors that can be applied to the present invention include: 4-(4-bromo-2-fluoroanilino)-6-methoxy-7-(1-methylpiperidin-4-ylmethoxy)quinazoline (generic name: vandetanib; a compound targeting VEGFR, EGFR and RET); 4-[4-[3-[4-Chloro-3-(trifluoromethyl)phenyl]ureido]phenoxy]-N-methylpyridine-2-carboxamide (generic name: sorafenib; a compound targeting BRAF, RET, etc.); N-[2-(Diethylamino)ethyl]-5-[(Z)-(5-fluoro-2-oxo-1,2-dihydro-3H-indol-3-ylidene)methyl]-2,4-dimethyl-1H-pyrrole-3-carboxamide mono[(2S)-2-hydroxysuccinate] (generic name: sunitinib; a compound targeting PDGFR, VEGFR, RET, etc.); N-(3,3-Dimethylindolin-6-yl)-2-(pyridin-4-ylmethylamino)nicotinamide (generic name: motesanib; a compound targeting PDGFR, VEGFR, RET, etc.); and N-(4-(6,7-dimethoxyquinolin-4-yloxy)phenyl)-N-(4-fluorophenyl)cyclopropane-1,1-dicarboxamide (generic name: XL184/ cabozantinib; a compound targeting MET, RET, etc.).

The definition of the term "sample", the method for extracting a DNA, an RNA or the like from the sample, the procedure for detecting the presence or absence of the CEP55-RET fusion polynucleotide or the CEP55-RET fusion polypeptide, and other related information are as described above.

If the presence of the CEP55-RET fusion polynucleotide or the CEP55-RET fusion polypeptide in a sample isolated from a patient is detected according to the inventive method, the patient will be determined to be highly responsive to a cancer treatment with a RET tyrosine kinase inhibitor. If the presence of the polynucleotide or polypeptide is not detected, the patient will be determined to be less responsive to a cancer treatment with a RET tyrosine kinase inhibitor.

### <Agents for detecting the presence or absence of the CEP55-RET fusion polynucleotide or the CEP55-RET fusion polypeptide, and agents for determining the effectiveness of cancer treatments with a RET tyrosine kinase inhibitor>

As described above, the polynucleotides noted below in (a) to (c), which each have a chain length of at least 15 nucleotides, can be used advantageously for detecting the presence or absence of the CEP55-RET fusion polynucleotide. Thus, said polynucleotides can also be used advantageously for determining the effectiveness of cancer treatments with a RET tyrosine kinase inhibitor.
(a) A polynucleotide or polynucleotides that are at least one probe selected from the group consisting of a probe that hybridizes to a polynucleotide encoding CEP55 protein and a probe that hybridizes to a polynucleotide encoding RET protein;
(b) a polynucleotide that is a probe that hybridizes to a point of fusion between a polynucleotide encoding CEP55 protein and a polynucleotide encoding RET protein; and
(c) polynucleotides that are a pair of primers designed to sandwich a point of fusion between a polynucleotide encoding CEP55 protein and a polynucleotide encoding RET protein.

Said polynucleotides each have a nucleotide sequence complementary to a particular nucleotide sequence of a target gene. As referred to herein, the term "complementary" may not necessarily refer to perfect complementarity as long as hybridization is achieved. Said polynucleotides have generally at least 80% homology, preferably at least 90% homology, more preferably at least 95% homology, and particularly preferably 100% homology with such a particular nucleotide sequence.

The polynucleotides of (a) to (c) may be a DNA or a RNA, or may be such that part or all of the nucleotides are substituted by an artificial nucleic acid such as PNA (polyamide nucleic acid: a peptide nucleic acid), LNA™ (Locked Nucleic Acid; a bridged nucleic acid), ENA® (2'-O,4'-C-Ethylene-bridged Nucleic Acid), GNA (glycerol nucleic acid) or TNA (threose nucleic acid).

As described above, the antibody binding to an CEP55-RET fusion polypeptide can be used advantageously for detecting translation products (CEP55-RET fusion polypeptides) of the CEP55-RET fusion polypeptide.

The agents of the present invention can contain not only the foregoing substance (e.g., polynucleotide, antibody) as an active ingredient but also other pharmacologically acceptable components. Such other components include buffer agents, emulsifying agents, suspending agents, stabilizing agents, antiseptic agents, and physiological saline. As buffer agents, there can be used phosphates, citrates, acetates and the like. As emulsifying agents, there can be used gum arabic, sodium alginate, tragacanth, and the like. As suspending agents, there can be used glyceryl monostearate, aluminum monostearate, methylcellulose, carboxymethyl cellulose, hydroxymethyl cellulose, sodium lauryl sulfate, and the like. As stabilizing agents, there can be used propylene glycol, diethylene sulfite, ascorbic acid, and the like. As antiseptic agents, there can be used sodium azide, benzalkonium chloride, paraoxybenzoic acid, chlorobutanol, and the like.

A specimen containing the inventive polynucleotide or antibody may also be combined with other specimens such as a substrate required for detecting a label attached to the polynucleotide or the antibody, a positive control (e.g., CEP55-RET fusion polynucleotide, CEP55-RET fusion polypeptide, or cells bearing the same), a negative control, a counterstaining reagent for use for *in situ* hybridization or the like (e.g., DAPI), a molecule required for antibody detection (e.g., secondary antibody, Protein G, Protein A), and a buffer solution for use in sample dilution or washing; by combining such specimens, a kit for use in the method of the present invention can be provided. The inventive kit can contain instructions for use thereof. Therefore, the present invention also provides the foregoing kit for use in the inventive method.

### <Method for treatment of cancer, and therapeutic agents for cancer>

As described above, if the presence of the CEP55-RET fusion polynucleotide or the CEP55-RET fusion polypeptide is detected in a patient by the method of the present invention, the patient is considered to be highly responsive to a cancer treatment with a RET tyrosine kinase inhibitor. Thus, efficient cancer treatment is possible by administering a RET tyrosine kinase inhibitor selectively to those cancer patients who carry the CEP55-RET fusion gene. Therefore, this invention provides a method for treatment of cancer, comprising the step of administering a RET tyrosine kinase inhibitor to a patient in whom a cancer treatment with the RET tyrosine kinase inhibitor has been determined to be highly effective according to the foregoing determination method of this invention.

Further, the present invention provides a therapeutic agent for cancer, comprising a RET tyrosine kinase inhibitor as an active ingredient, wherein the therapeutic agent is to be administered to a patient in whom a cancer treatment with the RET tyrosine kinase inhibitor has been determined to be highly effective according to the foregoing determination method of this invention.

As described above, the "RET tyrosine kinase inhibitor" is not particularly limited as long as it is a substance capable of directly or indirectly suppressing the function of RET protein. Examples of known RET tyrosine kinase inhibitors that can be applied to the present invention are as given above.

The dosage form for administering a RET tyrosine kinase inhibitor to a patient is selected as appropriate depending on various factors including the type of the inhibitor and the type of cancer, and examples of the dosage form that can be adopted include oral, intravenous, intraperitoneal, transdermal, intramuscular, intratracheal (aerosol), rectal, intravaginal and other administrations.

### EXAMPLES

Hereunder, the present invention will be more specifically described on the basis of Examples, but this invention is not limited to the examples given below.

### <Test samples>

Thirteen surgically resected and frozen specimens of diffuse-type gastric cancer (DGC) were used as an object to be tested. As a negative control, normal gastric mucosal tissues were also used.

### <RNA extraction>

The tumor tissues cryopreserved in liquid nitrogen were pulverized with cryoPREP (product name: CP02; Covaris). Then, total RNA was extracted from the pulverized tumor tissues using a total RNA purification kit (product name: RNAeasy; QIAGEN).

### <RNA sequencing>

Library synthesis was performed with an mRNAseq sample preparation kit (Illumina) using 2 µg of the total RNA prepared hereinabove. More specifically, 2 µg of the total RNA was fragmented by treatment at 94°C for 5 minutes and subjected to cDNA synthesis. Next, a sequencing adapter was ligated to each end of the resulting cDNA, which was then subjected to electrophoresis on agarose gel, followed by purification. PCR was performed using the purified cDNA as a template to construct a 300 bp cDNA library. The sequences of 50 bp from both ends of the constructed cDNA library were sequenced using a high-throughput sequencer (GA2X; Illumina).

### <Analysis of sequence information>

The obtained sequence information, from which overlapping clones generated by PCR were excluded, was mapped to known databases (Refseq and Ensemble) using the Bowtie software to extract clones whose end sequences are derived from different genes (refer to Non-patent Document 4).

### <Verification by RT-PCR and Sanger method>

The same total RNA as used in RNA sequencing was subjected to cDNA synthesis anew using a reverse transcriptase (SuperScript III First-Strand Synthesis System; Invitrogen). PCR primers were synthesized based on the resulting sequences. PCR was performed using ExTaq HS (Takara), and then the PCR products were verified by electrophoresis. Further, the PCR products were extracted from the agarose gel and sequenced by the Sanger method using the same primers, the BigDye Terminator v3.1 Cycle Sequencing Kit (Applied Biosystems) and the ABI 3730 Sequencer. The obtained results were used to identify points of fusion of fusion genes and reading frames.

The conditions for reverse transcription reaction and PCR are as follows.

### <Reverse transcription reaction>

First, 5 µg (8 µL) of the total RNA was mixed with 1 µL of Random Hexamer Primer (50 ng/µL) and 1 µL of 10mM dNTP Mix, and the mixture was reacted at 65°C for 5 minutes and quenched on ice. Next, 2 µL of 10×RT buffer, 4 µL of 25 mM MgCl₂, 2 µL of 0.1 M DTT, 1 µL of RNaseOUT (40 U/µL), and 1 µL of SuperScript III RT (200 U/µL) were added in this order, and reverse transcription reaction was effected under the following conditions: 25°C for 10 minutes, 50°C for 50 minutes, 85°C for 5 minutes, and 4°C for 5 minutes. To the resulting reverse transcripts, 1 µL of RNaseH was added to digest the total RNA at 37°C over 20 minutes. The completed reverse transcription reactions were stored at -20°C until use.

### <PCR>

First, 2 µL of the hereinabove prepared 1st strand cDNA, 1 µL of 10×ExTaq buffer, 1.2 µL of 2.5 mM dNTPs, 4.7 µL of H₂O, 0.1 µL of ExTaq HS, and 1 µL of 2 µM CF/CR primer pair were mixed, and the mixture was subjected to PCR under the following conditions: the reaction started with 95°C for 3 minutes, followed by 35 cycles consisting of 94°C for 30 seconds, 58°C for 30 seconds, and 72°C for 30 seconds, and ended with 72°C for 5 minutes.

### (Example 1)

### [Identification of novel kinase fusion genes by RNA sequencing]

From each of 13 clinical DGC specimens, there were obtained at least 8.0 × 10⁷ paired nucleotide sequences, with overlapping clones generated by PCR being excluded. As the result of comparison of these sequences to existing gene databases, the following two candidates for fusion gene between the CEP55 gene and the RET gene, as shown in FIG. 2, were detected each in one specimen: a polynucleotide encoding a polypeptide in which CEP55 protein and RET9 protein are fused together, and a polynucleotide encoding a polypeptide in which CEP55 protein and RET51 protein are fused together (hereinafter also referred to the "CEP55-RET fusion gene").

### [Verification by RT-PCR and Sanger sequencing]

Next, the same specimens were verified for the presence of the fusion gene of interest by RT-PCR and Sanger sequencing. As a result, RT-PCR revealed that specimen-specific amplification of said fusion gene was observed, or in other words that said fusion gene was expressed only in DGC tissues and not in normal gastric mucosal tissues.

Further, sequencing of the obtained PCR products revealed that, as shown in FIG. 2 and SEQ ID NOs: 7 and 9, in the CEP55-RET fusion gene, exon 3 in the CEP55 gene (i.e., the polynucleotide consisting of the nucleotide sequence of positions 488 to 763 in SEQ ID NO: 1, 7 or 9) and exon 12 in the RET gene (i.e., the polynucleotide consisting of the nucleotide sequence of positions 2327 to 2474 in SEQ ID NO: 3 or 5) are directly bound together, whereby the two genes are fused together without inconsistency in their reading frames.

Accordingly, the results presented hereinabove suggested that since the CEP55 and RET genes are present in the same chromosome 10 in the same orientation, these genes are fused by reciprocal translocation between two chromosomes 10 (refer to the left half of FIG. 1) or by breakage and reunion in chromosome 10 (refer to the right half of FIG. 1) in a specific manner to cancer cells such as DGC. In fact, analysis of the nucleotide sequences of the fusion sites in genomic DNAs obtained from cancer tissues from undifferentiated gastric cancer patients demonstrated that in the cancer tissues from said patients, the CEP55 and RET genes are fused together by breakage and reunion in chromosome 10.

### (Example 2)

### [Analyses of the function of the CEP55-RET fusion polypeptides, and of the effectiveness of RET tyrosine kinase inhibitors against cancer cells expressing said polypeptides]

It is considered that the above-mentioned gene fusion induces activation of RET protein, and that this activation induces activation of a downstream signal, thereby causing canceration of cells. Therefore, it is conceivable that RET tyrosine kinase inhibitors may be therapeutically effective in patients with such activations. In order to verify these points, analysis of the CEP55-RET fusion gene was made by following appropriate conventional methods, as described below.

First, a cDNA encoding a FLAG epitope tag was joined to the 5' end of the cDNA of the CEP55-RET fusion gene (a polynucleotide encoding a polypeptide in which CEP55 and RET51 are fused together) obtained from undifferentiated gastric cancer patients' cancer tissues, by aligning their translation reading frames with each other. The resulting product was cloned into the pMXs retroviral vector.

Also, site-directed mutagenesis was performed on this vector to construct a vector encoding a kinase activity mutant with substitution of one amino acid in a RET kinase region (KD-mutated CEP55-RET fusion polypeptide: K758M).

Next, normal murine fibroblast line NIH-3T3 cells were infected with each of the thus-prepared retroviral vectors to obtain cell lines stably expressing the wild-type or the mutated CEP55-RET fusion polypeptide.

Furthermore, a retroviral vector encoding a RET fusion gene detected in lung adenocarcinoma (fusion gene between the KIF5B gene and the RET gene; hereinafter also referred to as the "KIF5B-RET fusion gene"), as well as a retroviral vector encoding a kinase activity mutant with substitution of lysine for methionine in a RET kinase region, were constructed by the same procedures as described above. NIH-3T3 cells were infected with each of these retroviral vectors to prepare cell lines stably expressing the wild-type or the mutated KIF5B-RET fusion polypeptide. These cell lines were subjected to the tests described below as a control group.

As for the KIF5B-RET fusion gene, reference should be made to Kohno T., et al., Nature Medicine, published online on February 12, 2012, vol. 18, no. 3, p. 375-377. The "KIF5B-RET fusion variant 1" as referred to therein is the KIF5B-RET fusion gene which was used as a control in this working example.

In this specification, the CEP55-RET fusion gene and the KIF5B-RET fusion gene (or the CEP55-RET fusion polypeptide and the KIF5B-RET fusion polypeptide) are also collectively referred to as the "RET fusion genes (or RET fusion polypeptides)".

The cell lines stably expressing the wild-type or the mutated RET fusion polypeptide were each seeded in a soft agar medium (at an agar concentration in medium of 4 mg/mL; the same applies hereunder) and evaluated for their anchorage-independent colony-forming ability to thereby investigate the transforming ability of the RET fusion polypeptides. The results are shown in FIG. 5.

Further, the cell lines stably expressing the wild-type RET fusion polypeptides were each seeded in a soft agar medium supplemented with a low-molecular-weight RET tyrosine kinase inhibitor (vandetanib or XL184) and evaluated for their anchorage-independent colony-forming ability to thereby investigate the effect of the RET tyrosine kinase inhibitors against transformation induced by the RET fusion polypeptides. The results are shown in FIG. 6.

In addition, the cell lines stably expressing the wild-type or the mutated RET fusion polypeptide were each cultured in a liquid medium, subjected to serum starvation, and then cultured again with the medium being replaced with the one supplemented with a RET tyrosine kinase inhibitor (vandetanib or XL184). Protein from each of the thus-obtained cell lines was extracted and subjected to Western blotting analysis using antibodies against various phosphorylated or unphosphorylated proteins to thereby analyze the downstream signals of the RET fusion polypeptides. The results are shown in FIG. 7.

Furthermore, the cell lines stably expressing the wild-type or the mutated CEP55-RET fusion polypeptide, and the cell line stably expressing the wild-type KIF5B-RET fusion polypeptide, were each subcutaneously transplanted into immunodeficient mice (BALB/c-nu/nu) at a dose of 1 × 10⁶ cells per spot to thereby investigate the *in vivo* tumorigenic ability of the cells expressing these RET fusion polypeptides. The results are shown in FIG. 8.

As is evident from the results shown in FIG. 5, NIH-3T3 cells showed anchorage-independent colony formation due to the expression of the CEP55-RET fusion polypeptide. On the other hand, it was found that the anchorage-independent colony-forming ability of the CEP55-RET fusion polypeptides is significantly suppressed by inactivating the kinase activity of RET protein.

It was also found that, as shown in FIG. 6, the anchorage-independent colony-forming ability of the CEP55-RET fusion polypeptides is significantly suppressed by using a RET tyrosine kinase inhibitor.

As is evident from the results shown in FIG. 7, NIH-3T3 cells showed strong phosphorylation of the RET kinase domain due to the expression of the CEP55-RET fusion polypeptide (refer to the lanes labeled as "W" in the "p-RET (Y1062)" row of FIG. 7). It was also found that this phosphorylation is significantly suppressed by treatment with a RET tyrosine kinase inhibitor or by inactivation of the kinase activity of RET protein (refer to the lanes labeled as "V" and "X", or "KD" in the "p-RET (Y1062)" row of FIG. 7).

As shown in FIG. 7, the phosphorylation of AKT was strongly induced by the CEP55-RET fusion polypeptide (refer to the lanes labeled as "W" in the "p-AKT (S473)" row of FIG. 7). It was also found that phosphorylation of STAT3 and MAPK is increased by said fusion peptide (refer to the lanes labeled as "W" in the "p-STAT3 (Y705)" and "p-MAPK (T202/Y204705)" rows of FIG. 7), but that the phosphorylation of these factors is also significantly suppressed by treatment with a RET tyrosine kinase inhibitor or by inactivation of the kinase activity of RET protein (refer to the lanes labeled as "V" and "X", or "KD" in the "p-RET (Y1062)", "p-STAT3 (Y705)" and "p-MAPK (T202/Y204705)" rows of FIG. 7).

As shown in FIG. 8, tumorigenesis was observed within 14 days after NIH-3T3 cells expressing the wild-type CEP55-RET fusion polypeptide were subcutaneously transplanted into immunodeficient mice. On the other hand, NIH-3T3 cells expressing the mutated CEP55-RET fusion polypeptide were also subcutaneously transplanted into immunodeficient mice, but no tumorigenesis was observed at all for 30 days after the transplantation.

Accordingly, these findings demonstrated that the CEP55-RET fusion gene serves as an oncogene with transforming ability, and that this transformation requires the activation of the RET kinase activity.

It is also found that the transforming ability of the CEP55-RET fusion polypeptide is suppressed using a RET tyrosine kinase inhibitor by inhibiting the activations of the RET tyrosine kinase in said fusion polypeptide and its downstream signals such as AKT.

### INDUSTRIAL APPLICABILITY

As described above, the present invention enables detection of polynucleotides encoding fusion polypeptides between CEP55 protein or part thereof and RET protein or part thereof, as well as expression products of said polynucleotides, and also this detection makes it possible to predict the effectiveness of cancer treatments with a RET tyrosine kinase inhibitor. This fusion induces activation of RET protein, thereby causing canceration of cells. This fusion induces the activation of RET protein, and in turn causes canceration of cells. Further, as demonstrated above, said RET activation and canceration can be significantly suppressed by using a RET tyrosine kinase inhibitor. Therefore, since the fusion between the CEP55 gene and the RET gene can be targeted by RET tyrosine kinase inhibitors, the present invention is very useful in improving the efficiency of cancer treatments.

## Claims

1. A polynucleotide encoding a polypeptide in which CEP55 protein or part thereof and RET protein or part thereof are fused together.

2. A polypeptide encoded by the polynucleotide according to claim 1.

3. A method for detecting the presence or absence in a sample of the polynucleotide according to claim 1 or of the polypeptide according to claim 2, the method comprising the steps of:
(a) contacting the sample with an agent intended for specifically detecting the presence or absence of the polynucleotide or the polypeptide in the sample; and
(b) detecting the presence or absence of the polynucleotide or the polypeptide.

4. An agent for detecting the presence or absence in a sample of the polynucleotide according to claim 1 or of the polypeptide according to claim 2 by the method according to claim 3, the agent comprising a polynucleotide or polynucleotides as set forth below in any one of (a) to (c), the polynucleotide or polynucleotides having a chain length of at least 15 nucleotides, or an antibody as set forth below in (d):
(a) a polynucleotide or polynucleotides that are at least one probe selected from the group consisting of a probe that hybridizes to a polynucleotide encoding CEP55 protein and a probe that hybridizes to a polynucleotide encoding RET protein;
(b) a polynucleotide that is a probe that hybridizes to a point of fusion between a polynucleotide encoding CEP55 protein and a polynucleotide encoding RET protein;
(c) polynucleotides that are a pair of primers designed to sandwich a point of fusion between a polynucleotide encoding CEP55 protein and a polynucleotide encoding RET protein; and
(d) an antibody that binds to a polypeptide in which CEP55 protein and RET protein are fused together.

5. A method for determining the effectiveness of a cancer treatment with a RET tyrosine kinase inhibitor, the method comprising the step of detecting the presence or absence in a sample isolated from a patient of the polynucleotide according to claim 1 or of the polypeptide according to claim 2, wherein in a case where the presence of the polynucleotide or the polypeptide is detected, the cancer treatment with the RET inhibitor is determined to be highly effective in the patient.

6. An agent for determining the effectiveness of a cancer treatment with a RET inhibitor by the method according to claim 5, the agent comprising a polynucleotide or polynucleotides as set forth below in any one of (a) to (c), the polynucleotide or polynucleotides having a chain length of at least 15 nucleotides, or an antibody as set forth below in (d):
(a) a polynucleotide or polynucleotides that are at least one probe selected from the group consisting of a probe that hybridizes to a polynucleotide encoding CEP55 protein and a probe that hybridizes to a polynucleotide encoding RET protein;
(b) a polynucleotide that is a probe that hybridizes to a point of fusion between a polynucleotide encoding CEP55 protein and a polynucleotide encoding RET protein;
(c) polynucleotides that are a pair of primers designed to sandwich a point of fusion between a polynucleotide encoding CEP55 protein and a polynucleotide encoding RET protein; and
(d) an antibody that binds to a polypeptide in which CEP55 protein and RET protein are fused together.

7. A method for treatment of cancer, comprising the step of administering a RET tyrosine kinase inhibitor to a patient in whom a cancer treatment with the RET tyrosine kinase inhibitor has been determined to be highly effective by the method according to claim 5.

8. A therapeutic agent for cancer, comprising a RET tyrosine kinase inhibitor as an active ingredient, wherein the therapeutic agent is to be administered to a patient in whom a cancer treatment with the RET tyrosine kinase inhibitor has been determined to be highly effective by the method according to claim 5.
